# EUROPEAN PATENT APPLICATION

(11) **EP 4 411 377 A1**
(43) Date of publication of application: **07.08.2024**
(21) Application number: 22875465.1
(22) Date of filing: 10.06.2022
(51) Int. Cl.: G01N 33/543, G01N 21/76

(54) **IMMUNOCHROMATOGRAPHY KIT AND TESTING DEVICE**

(30) Priority: 29.09.2021 JP 2021159997
(71) Applicant: FUJIFILM Corporation, Tokyo 106-8620 (JP)
(72) Inventor: ITO, Yukino, Ashigarakami-gun, Kanagawa 258-8538 (JP)
(74) Representative: Hughes, Andrea Michelle
(86) International application number: PCT/JP2022/023441
(87) International publication number: WO 2023/053586

(57) **Abstract**

The present invention relates to an immunochromatography kit and an assay apparatus. The immunochromatography kit includes a cartridge that accommodates an assay strip in which a sample is spread and which has an assay region where a test substance contained in the sample is captured, a binding substance for capturing that specifically binds to the test substance, where the binding substance for capturing is labeled with a luminescent protein and immobilized on the assay region, a labeled binding substance that specifically binds to the test substance, where the labeled binding substance is labeled with a fluorescent substance and supplied to the assay region, and a luminescent substrate that is supplied to the assay region, where the luminescent substrate causes a phenomenon of bioluminescence resonance energy transfer, in which the luminescent substrate reacts with the luminescent protein to generate energy, and the energy is transferred to the fluorescent substance in a state where the labeled binding substance is captured by the binding substance for capturing through the test substance.

## Description

### BACKGROUND OF THE INVENTION

### 1. Field of the Invention

The present disclosure relates to an immunochromatography kit and an assay apparatus.

### 2. Description of the Related Art

An immunological measuring method is widely used as a method of qualitatively or quantitatively measuring a test substance present in a biological specimen such as urine or blood. Among the above, immunochromatography is highly convenient since the operation is easy and measurement can be carried out in a short time.

In the immunochromatography, an assay strip, on which an antibody (a capture antibody) that specifically binds to a test substance (for example, an antigen) is immobilized in an assay region, is used. As an assay method that is used in the immunochromatography, a sandwich method and a competition method are widely used. In the sandwich method, an antibody subjected to labeling (a labeled antibody), which specifically binds to an antigen, and a sample are spread on an assay strip to form a composite body of the capture antibody-antigen-labeled antibody in the assay region in a case where the antigen is contained in the sample. Then, a signal such as color development due to the labeling of the labeled antibody is detected to qualitatively or quantitatively measure the antigen which is a test substance. On the other hand, in the competition method, a pseudo antigen subjected to labeling (a labeled pseudo antigen) and a sample solution are spread on an assay strip, and an antigen in the sample solution and the labeled pseudo antigen are competitively captured by a capture antibody in the assay region. Then, a signal such as color development due to the labeling of the labeled pseudo antigen captured in the assay region is detected to qualitatively or quantitatively measure the antigen which is a test substance. In the sandwich method, the signal intensity due to the labeling increases as the antigen amount in the sample increases, whereas in the competition method, the signal intensity due to the labeling decreases as the antigen amount in the sample increases.

In such immunochromatography as described above, there is known chemiluminescence immunochromatography in which a luminescent protein is used as a label and the luminescence due to the luminescent protein, which occurs by subjecting the luminescent protein and a luminescent substrate to a chemical reaction, is detected (for example, JP2015-105858A). In the chemiluminescence immunochromatography, a sample and a labeled antibody labeled with a luminescent protein are spread on an assay strip on which a capture antibody is immobilized in an assay region. Then, a luminescent substrate, which reacts with the luminescent protein as a label to generate light, is spread on the assay strip. The luminescence, which occurs by the reaction between the luminescent protein captured through the labeled antibody captured in the assay region and the luminescent substrate, is detected to qualitatively or quantitatively measure the antigen which is a test substance.

In addition, JP2018-522221A discloses chemiluminescence immunochromatography that uses a competition method, where a technique referred to as bioluminescence resonance energy transfer (BRET) that occurs between a luminescent protein and a fluorescent substance is used. In JP2018-522221A, an assay strip on which a capture antibody labeled with a luminescent protein is immobilized in an assay region is used, and a sample, a labeled pseudo antigen labeled with a fluorescent substance prepared in advance, and a luminescent substrate that causes the luminescent protein to undergo a reaction are spread on the assay strip. Then, an antigen in the sample and the labeled pseudo antigen are competitively captured by the capture antibody. In the assay region, in a case where the luminescent protein and the fluorescent substance are close to each other, the energy generated by the reaction between the luminescent substrate and the luminescent protein is transferred to the fluorescent substance to cause fluorescence to be generated. In the chemiluminescence immunochromatography described in JP2018-522221A, the amount of the labeled pseudo antigen captured in the assay region decreases as the amount of the antigen in the sample captured increases, and thus the fluorescence due to BRET decreases. In the assay region, this fluorescence due to BRET is detected to qualitatively or quantitatively measure the antigen which is a test substance.

### SUMMARY OF THE INVENTION

In the case of JP2015-105858A, the labeled antibody labeled with a luminescent protein is spread in the entire region including the assay region in the assay strip. Among these, a part of the labeled antibodies specifically binds to an antigen by an antigen-antibody reaction; however, a part of the remaining labeled antibodies may remain in the periphery of the assay region even though they do not specifically bind to the antigen. In a case where the luminescent substrate is spread on the assay strip, the luminescent protein, which is not only the label of the labeled antibody specifically adsorbed to the antigen but also the label of the remaining labeled antibody, reacts with the luminescent substrate. In a case where the peripheral region of the assay region is set as a background, the luminescence due to the remaining labeled antibody occurs even in the background. Since a light signal in the background becomes noise with respect to a light signal generated by the antigen-antibody reaction, the signal (S)/noise (N) ratio decreases, and thus the measurement accuracy decreases.

In JP2018-522221A, the luminescent protein is immobilized only in the assay region as a label of the capture antibody. Therefore, fluorescence is generated due to BRET only in the assay region in which the luminescent protein of the capture antibody and the fluorescent substance of the labeled antibody come close to each other. As a result, no fluorescence is generated in the background in which the capture antibody is not present. However, in the competition method that is used in JP2018-522221A, the amount of the labeled pseudo antigen that binds to the capture antibody immobilized in the assay region is maximized in a state where an antigen is not present in the sample. Therefore, the signal intensity is highest in a state where the antigen is not present, and the signal intensity decreases as the antigen amount increases. That is, the signal intensity is maximized in a state where the antigen is not present, which serves as the baseline of the signal intensity for measuring the antigen amount; however, there is a problem that the signal intensity of the baseline fluctuates due to various factors. This is because the amount of the labeled pseudo antigen that binds to the capture antibody in a state where an antigen is not present in the sample changes due to the fluctuation of the fluid velocity that is caused by, for example, the force or the sample amount spotted in a case where the sample is spotted on the assay strip. In addition, in BRET, the signal intensity of fluorescence changes depending on the distance between the luminescent protein and the fluorescent substance. Therefore, in a case where there is a fluctuation in the binding state of the labeled pseudo antigen that binds to the capture antibody, the baseline also fluctuates. In a case where the baseline fluctuates, the measurement accuracy decreases. In particular, the smaller the antigen amount in the sample is, that is, the lower the antigen concentration in the sample is, the more the measurement accuracy is affected by the fluctuation of the signal intensity at each baseline measurement.

In an initial stage of infection with an influenza or the like in an infectious disease, the amount of the virus contained in a sample is very small. Therefore, there is a demand for an immunochromatography kit and an assay apparatus, which make it possible to detect a test substance even in a case where the test substance has a very small amount.

The present disclosure has been made in consideration of the above circumstances, and an object of the present disclosure is to provide an immunochromatography kit and an assay apparatus, which makes it possible to carry out a highly accurate measurement even in a case where the concentration of a test substance in a sample is low, as compared with those in the related art.

An immunochromatography kit according to the present disclosure includes;
a cartridge that accommodates an assay strip in which a sample is spread and which has an assay region where a test substance contained in the sample is captured,
a binding substance for capturing that specifically binds to the test substance, where the binding substance for capturing is labeled with a luminescent protein and immobilized on the assay region,
a labeled binding substance that specifically binds to the test substance, where the labeled binding substance is labeled with a fluorescent substance and supplied to the assay region, and
a luminescent substrate that is supplied to the assay region, where the luminescent substrate causes a phenomenon of bioluminescence resonance energy transfer, in which the luminescent substrate reacts with the luminescent protein to generate energy, and the energy is transferred to the fluorescent substance in a state where the labeled binding substance is captured by the binding substance for capturing through the test substance.

The luminescent protein may be such that it is a luminescent protein that causes blue luminescence to occur, and the fluorescent substance is a green fluorescent dye or a green fluorescent protein, which emits green fluorescence having a quantum yield of 0.7 or more.

The luminescent protein may be such that it is a blue luminescent protein that causes blue luminescence to occur, and the fluorescent substance has a peak wavelength of 530 nm or more.

A sample collection tool for collecting the sample may be included.

An instruction manual that describes an assay procedure of supplying the sample and the labeled binding substance to the assay region and then supplying the luminescent substrate to the assay region may be included.

A label holding pad containing the labeled binding substance may be disposed at a supply position of the sample on the assay strip.

A luminescent substrate solution holding part that encompasses a substrate solution containing the luminescent substrate may be provided in a cartridge.

A washing solution, which is supplied to the assay region after supplying the sample and the labeled binding substance to the assay region and before supplying the luminescent substrate to the assay region, where the washing solution is used to wash the labeled binding substance that has non-specifically adsorbed in the assay region, may be further provided.

A washing solution holding part that encompasses the washing solution may be provided in the cartridge.

The immunochromatography assay apparatus according to the present disclosure includes;
a loading unit in which the cartridge in the immunochromatography kit according to the present disclosure is attachably and detachably loaded,
a detection unit that detects an intensity of luminescence from the fluorescent substance in the assay region, and
a processor that is configured to determine whether the sample is positive or negative based on the intensity of the luminescence acquired from the detection unit

The assay apparatus according to the present disclosure may be such that it includes a luminescent substrate supply mechanism that supplies the luminescent substrate to the assay region, where the processor is configured to supply the luminescent substrate to the assay region by the luminescent substrate supply mechanism after the sample and the labeled binding substance are supplied to the assay region.

The assay apparatus according to the present disclosure may be such that it further includes;
a washing solution supply mechanism that supplies the washing solution to the assay region,
where the cartridge having a washing solution holding part in the inside thereof is loaded into the loading unit, and
the processor is configured to supply the washing solution to the assay region by the washing solution supply mechanism after supplying the sample and the labeled antibody to the assay region and before supplying the luminescent substrate to the assay region.

The assay apparatus according to the present disclosure may be such that the detection unit detects the intensity of the luminescence from the fluorescent substance (the luminescence intensity of the fluorescent substance at a peak wavelength thereof) in the assay region as a first luminescence intensity and detects an intensity of luminescence from the luminescent protein (the luminescence intensity of the luminescent protein at a peak wavelength thereof) as a second luminescence intensity, and the processor is configured to acquire the first luminescence intensity and the second luminescence intensity from the detection unit and carry out the determination using a value obtained by dividing the first luminescence intensity by the second luminescence intensity.

The assay apparatus according to the present disclosure may be such that the detection unit detects, as a first luminescence intensity, the intensity of the luminescence from the fluorescent substance in the assay region and detects, as a third luminescence intensity, an intensity of luminescence at an isoluminescence point, which is present in a wavelength range between a peak wavelength of the luminescence from the fluorescent substance and a peak wavelength of the luminescence from the luminescent protein, and the processor is configured to acquire the first luminescence intensity and the third luminescence intensity from the detection unit and carry out the determination using a value obtained by dividing the first luminescence intensity by the third luminescence intensity.

According to the immunochromatography kit and the assay apparatus according to the present disclosure, it is possible to carry out a highly accurate measurement even in a case where the concentration of a test substance in a sample is low, as compared with those in the related art.

### BRIEF DESCRIPTION OF THE DRAWINGS

Fig. 1 is a view showing an overall configuration of an immunochromatography kit.
Fig. 2 is an exploded perspective view of a cartridge.
Fig. 3 is a view showing a procedure of an immunochromatography assay.
Fig. 4A is a view illustrating luminescence from an assay region in a negative case, and Fig. 4B is a view illustrating luminescence from an assay region in a positive case.
Fig. 5A is a view showing an example of an emission spectrum detected from an assay region in a negative case, and Fig. 5B is a view showing an example of an emission spectrum detected from an assay region in a positive case.
Fig. 6 is a view showing a relationship between a luminescence intensity of light generated from a fluorescent substance due to BRET and a concentration of a test substance.
Fig. 7 is a view showing a point of isoluminescence intensity in a negative case and a positive case.
Fig. 8 is a view showing luminescence from an assay region in chemiluminescence immunochromatography assay (a related art 1) that does not use BRET.
Regarding chemiluminescence immunochromatography assay (a related art 2) that uses a competition method and BRET, Fig. 9A is a view illustrating luminescence from an assay region in a negative case, and Fig. 9B is a view illustrating luminescence from an assay region in a positive case.
Fig. 10 is a view showing a relationship between a luminescence intensity of light generated from a fluorescent substance due to BRET and a concentration of a test substance in the related art 2.
Fig. 11 is a perspective view of a cartridge of a design change example.
Fig. 12 is an exploded perspective view of the cartridge of the design change example.
Fig. 13 is a broken side view of the cartridge of the design change example.
Fig. 14 is a broken side view of the cartridge, which shows a state where a first pressing operation part is pushed.
Fig. 15 is a broken side view of the cartridge, which shows a state where a second pressing operation part is pushed in addition to the first pressing operation part.
Fig. 16 is a perspective view showing an assay apparatus.
Fig. 17 is a cross-sectional view showing the inside of the assay apparatus.
Fig. 18 is a block diagram showing a processing unit of a CPU of the assay apparatus.
Fig. 19 is a flowchart showing a processing procedure of the assay apparatus.

### DESCRIPTION OF THE PREFERRED EMBODIMENTS

Hereinafter, specific embodiments of the immunochromatography kit and the assay apparatus according to the present disclosure will be described with reference to the drawings. The constitutional elements indicated by the same reference numerals in the drawings mean the same constitutional elements.

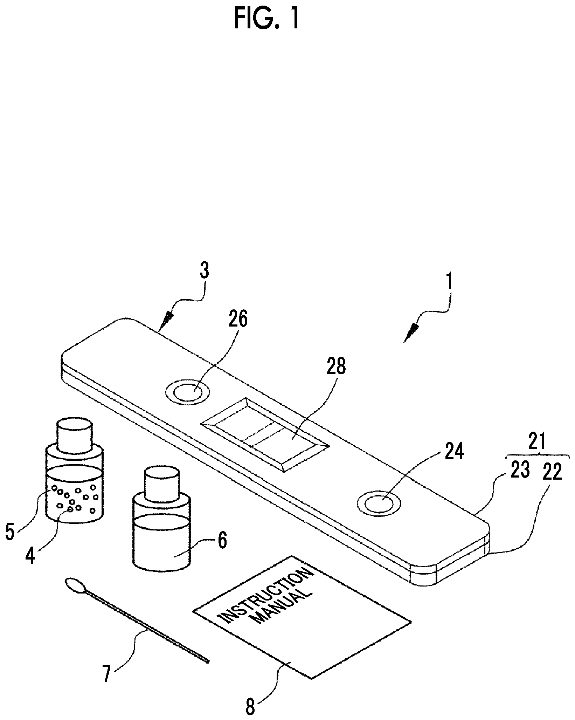

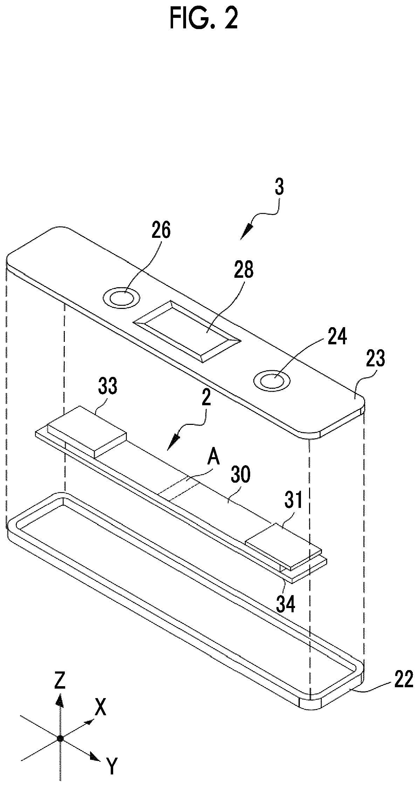

As shown in Fig. 1 and Fig. 2, an immunochromatography kit 1 according to one embodiment includes a cartridge 3 that accommodates an assay strip 2, a luminescent substrate solution 5 containing a luminescent substrate 4, a washing solution 6, a sample collection tool 7, and an instruction manual 8.

The cartridge 3 belongs to a single-use type that is used one by one for each sample, and it has a case 21 having an elongated rectangular parallelepiped shape. The case 21 is composed of a case main body 22 and a cover member 23. The case main body 22 has an elongated plate shape and includes a recessed part that accommodates the assay strip 2. The cover member 23 includes a sample dropping port 24, a luminescent substrate dropping port 26, and an observation window 28. It is noted that both the X direction and the Y direction in Fig. 2 are directions along the horizontal plane and are orthogonal to each other. The X direction is a direction along a short side of the case 21 and the Y direction is a direction along a long side of the case 21. The Z direction is a direction along the vertical direction and is orthogonal to the X direction and the Y direction. The same applies to the following drawings.

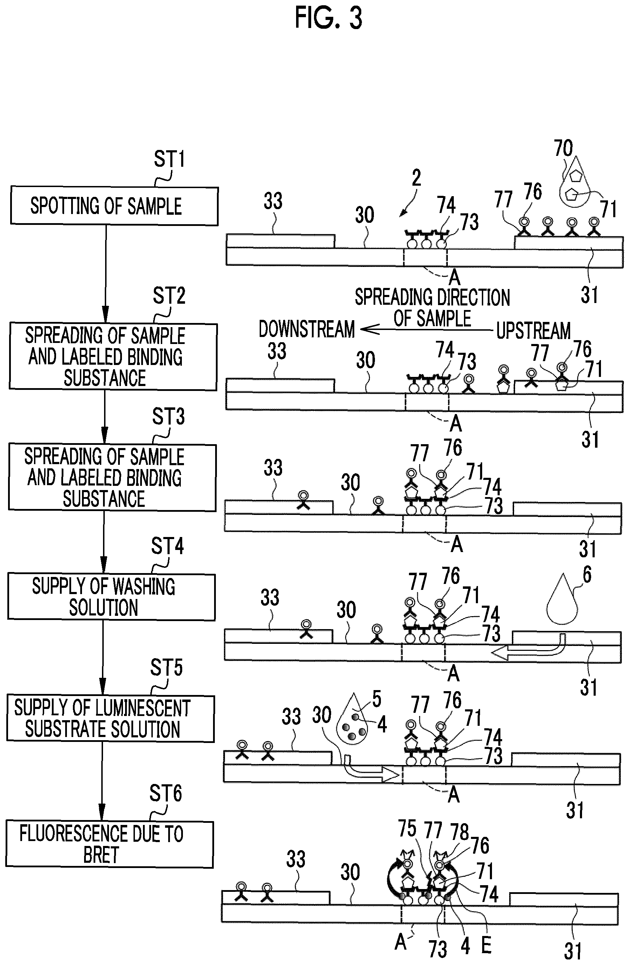

The sample dropping port 24 is a round hole to which a sample solution 70 (see Fig. 3) containing a sample is dropped. The sample may be any sample as long as it can contain a test substance 71, and it is not particularly limited. The sample includes a biological specimen of an individual as a target of the immunochromatography assay, particularly an animal as well as a human, for example, blood, serum, blood plasma, spinal fluid, tear fluid, sweat, urine, feces, pus, nasal discharge, nasal swab, pharynx swab, nasal aspirate, or sputum, as well as an organ, a tissue, a mucous membrane, and skin, or swabs containing these, or an animal or plant itself or a dried form thereof. Examples of the test substance 71 include an antigen, an antibody, a protein, and a low-molecular-weight compound.

It is noted that the washing solution 6 is also dropped from the sample dropping port 24.

The luminescent substrate dropping port 26 is a round hole to which the luminescent substrate solution 5 containing the luminescent substrate 4 is dropped. Here, the description that the luminescent substrate solution 5 is dropped has the same meaning as the description that the luminescent substrate 4 is dropped. The luminescent substrate 4 will be described later.

The observation window 28 is a rectangular opening for observing an assay region A of the assay strip 2 from the outside. The observation window 28 is formed between the sample dropping port 24 and the luminescent substrate dropping port 26.

The sample collection tool 7 is a tool for collecting a sample, and a swab for collecting a nasal swab is used in the present example. As the sample collection tool 7, an appropriate collection tool can be provided depending on the sample. It is noted that a swab in which a sample is collected is immersed in a spreading solution (also referred to as a sample treatment liquid), which is not illustrated in the drawing, and the collected sample is extracted to prepare the sample solution 70 (see Fig. 3). The sample solution 70 containing a sample, which is obtained as described above, is dropped from the sample dropping port 24. That is, the description that the sample solution 70 is added dropwise has the same meaning as the description that the sample is added dropwise.

The instruction manual 8 describes a method of using the assay strip 2, the luminescent substrate solution 5, the washing solution 6, the sample collection tool 7, and the like, as well as an assay procedure and the like. The instruction manual 8 describes an assay procedure of supplying the sample and the labeled binding substance to the assay region A and then supplying the luminescent substrate 4 to the assay region A. The details of the assay procedure will be described later. It is noted that the instruction manual 8 enclosed in the immunochromatography kit 1 may be a document which describes, in addition to a booklet which describes the assay procedure itself, an access code (URL: uniform resource locator (URL) or the like) for accessing, via a communication line, an assay procedure manual that is provided by being uploaded to a server in a form of electronic data.

The configuration and the function of the cartridge 3 will be described in detail with reference to Fig. 2 and Fig. 3. Fig. 2 is a view showing a configuration of the cartridge 3, and Fig. 3 is a view showing an assay procedure using the cartridge 3. As shown in Fig. 2, the assay strip 2 has an elongated rectangular plate shape as a whole and has a carrier 30, a label holding pad 31, an absorption pad 33, and a back pressure-sensitive adhesive sheet 34.

The carrier 30 is formed from a porous insoluble material such as a nitrocellulose membrane. The assay region A in which the test substance 71 (see Fig. 3) contained in the sample is captured is provided near the center of the carrier 30. A binding substance for capturing 74 (see Fig. 3) which is labeled with a luminescent protein 73 (see Fig. 3) is immobilized in the assay region A.

The binding substance for capturing 74 is a binding substance that specifically binds to the test substance 71. For example, in a case where the test substance 71 is an antigen, the binding substance for capturing 74 is an antibody against the antigen, and in a case where the test substance 71 is an antibody, the binding substance for capturing 74 is an antigen against the antibody. In a case where the test substance 71 is a protein, a low-molecular-weight compound, or the like, the binding substance for capturing 74 is an aptamer with respect to the protein, the low-molecular-weight compound, or the like.

The luminescent protein 73 that is labeled to the binding substance for capturing 74 is a protein that reacts with the luminescent substrate 4 to cause luminescence.

The label holding pad 31 is attached to the position of the carrier 30 that faces the sample dropping port 24. The sample solution 70 dropped onto the sample dropping port 24 is spotted on the label holding pad 31. That is, the label holding pad 31 is disposed at a sample supply position on the assay strip 2.

The label holding pad 31 contains a labeled binding substance 77 labeled with a fluorescent substance 76 (see Fig. 3). The labeled binding substance 77 is a binding substance that specifically binds to the test substance 71. For example, in a case where the test substance 71 is an antigen, the labeled binding substance 77 is an antibody against the antigen, and in a case where the test substance 71 is an antibody, the labeled binding substance 77 is a secondary antibody that can bind to the antibody. In a case where the test substance 71 is a protein, a low-molecular-weight compound, or the like, the labeled binding substance 77 is an aptamer with respect to the protein, the low-molecular-weight compound, or the like.

The binding substance for capturing 74 and the labeled binding substance 77 may be the same or different from each other. For example, in a case where the test substance 71 is an influenza A type virus or a biomarker thereof, it is possible to use an anti-influenza A type monoclonal antibody (manufactured by Medix Biochemica Inc., product name: Anti-influenza A SPT N-5 7307) as the binding substance for capturing 74 and the labeled binding substance 77.

The fluorescent substance 76 is a fluorescent dye, a fluorescent protein, or the like, which causes fluorescence to be generated in a case where excitation energy is applied.

As the luminescent protein 73 and the fluorescent substance 76, those having an overlap between the wavelength range of the emission spectrum of the luminescent protein 73 and the wavelength range of the excitation spectrum of the fluorescent substance 76 are selected. Energy transfer can occur in a case where there is a wavelength range in which the emission spectrum of the luminescent protein 73 overlaps with the excitation spectrum of the fluorescent substance 76.

The luminescent protein 73 is, for example, a luminescent protein that emits blue light. In this case, the fluorescent substance 76 having a quantum yield of 0.7 or more can be allowed to be a green fluorescent dye or a green fluorescent protein, which emits green fluorescence. Here, the blue light is light having a maximal luminescence wavelength (hereinafter, referred to as a peak wavelength) in a wavelength range of about 430 nm to about 490 nm, and the green light is light having a peak wavelength in a wavelength range of about 490 nm to about 550 nm. In addition, it is preferable that the difference between the peak wavelength of the luminescent protein 73 and the peak wavelength of the fluorescent substance 76 is 40 nm or more. As an example, in a case where the luminescent protein 73 is a blue luminescent protein that causes blue luminescence, the fluorescent substance 76 can be allowed to be a fluorescent dye or a fluorescent protein, which has a peak wavelength of 530 nm or more. The farther the peak wavelengths are separated, the more easily the peak of each light is identified, which is preferable. For example, in a case where the luminescent protein 73 has a peak wavelength in a wavelength range of blue light of about 430 nm to about 490 nm, the fluorescent substance 76 has a peak wavelength in a wavelength range of red light of about 640 nm to 770 nm.

The luminescent substrate 4 reacts with the luminescent protein 73 and then is oxidized to generate energy. This causes an excited state, and luminescence occurs in a case where a transition to the ground state occurs. On the other hand, in a case where the luminescent protein 73 and the fluorescent substance 76 are positioned to be close to each other, a phenomenon of bioluminescence resonance energy transfer (BRET), in which the energy generated by the reaction between the luminescent substrate 4 and the luminescent protein 73 is transferred to the fluorescent substance 76, occurs. As a result, the fluorescent substance 76 emits light. That is, due to the energy E of the luminescence that occurs by the reaction between the luminescent substrate 4 and the luminescent protein 73, the fluorescent substance 76 is excited to cause fluorescence to be generated from the fluorescent substance 76. In the assay using the immunochromatography kit 1 according to the present disclosure, it is determined whether a sample is positive or negative by detecting the luminescence from the fluorescent substance 76, which occurs due to BRET. The details of the assay procedure will be described later.

Specific examples of the combination of the luminescent protein 73, the fluorescent substance 76, and the luminescent substrate 4 include a combination of NanoLuc (registered trade name, manufactured by Promega Corporation) which is a blue luminescent protein, mNeongreen (manufactured by Allele Biotechnology and Pharmaceuticals, Inc.) which is a green fluorescent protein, and Furimazine (manufactured by Promega Corporation).

The absorption pad 33 is attached to one end on a side opposite to the other end of the carrier 30 to which the label holding pad 31 is attached. The absorption pad 33 is formed from a porous material similarly to the carrier 30. The absorption pad 33 absorbs the sample solution 70, the washing solution 6, and the luminescent substrate solution 5, which are spread on the carrier 30.

The back pressure-sensitive adhesive sheet 34 is a base material of which the surface is a pressure-sensitive adhesive surface, and the carrier 30 is adhesively fixed to the back pressure-sensitive adhesive sheet 34.

The washing solution 6 is supplied to the assay region A after the sample and the labeled binding substance 77 are supplied to the assay region A and before the luminescent substrate 4 is supplied to the assay region. The washing solution 6 is used to wash the labeled binding substance 77 that has non-specifically adsorbed in the assay region A. Here, the "non-specific adsorption" refers to a state where the labeled binding substance 77 is adsorbed on the carrier regardless of the specific binding between the test substance 71 and the binding substance for capturing 74.

Hereinafter, a procedure of the immunochromatography assay will be described with reference to Fig. 3. Here, a description will be made using, as an example, a case where a sample contains the test substance 71, that is, a case where the sample is positive. It is noted that in Fig. 3, the back pressure-sensitive adhesive sheet 34 is not illustrated in the drawing.

First, as shown in a step ST1, the sample solution 70 is spotted on the label holding pad 31. The test substance 71 in the sample solution 70, which has been spotted on the label holding pad 31, specifically binds to the labeled binding substance 77 of the label holding pad 31.

As shown in a step ST2, the sample solution 70 permeates into the carrier 30 from the label holding pad 31 and then is spread on the assay region A side (downstream side) by the capillary action. The test substance 71 that has reached the assay region A is captured by the binding substance for capturing 74. As a result, the fluorescent substance 76 is captured by the binding substance for capturing 74 of the assay region A through the test substance 71 and the labeled binding substance 77.

As shown in a step ST3, the labeled binding substance 77 that has not been captured in the assay region A is further spread to the absorption pad 33 on the downstream side. It is noted that a part of the labeled binding substances 77 may not be spread to the absorption pad 33 and may be non-specifically adsorbed on the carrier 30.

Next, as shown in a step ST4, the washing solution 6 is supplied. The washing solution 6 is supplied to the carrier 30 through the label holding pad 31 and then is spread on the assay region A side (downstream side) by the capillary action. By this spreading of the washing solution 6, the labeled binding substance 77 that has non-specifically adsorbed on the carrier is washed. In this case, the labeled binding substance 77 that has non-specifically adsorbed in the assay region A is allowed to flow to the absorption pad 33 together with the washing solution 6.

Thereafter, as shown in a step ST5, the luminescent substrate solution 5 is supplied. The luminescent substrate solution 5 is supplied to the carrier 30 from the downstream side of the assay region A and then is spread on the assay region A side (upstream side).

As shown in a step ST6, the luminescent substrate 4 contained in the luminescent substrate solution 5 that has reached the assay region A reacts with the luminescent protein 73 immobilized in the assay region A. BRET, in which the energy E generated by this reaction is transferred to the captured fluorescent substance 76, occurs, and then fluorescence 78 occurs from the fluorescent substance 76. By the luminescence from the assay region A, it is possible to determine whether the sample is positive or negative.

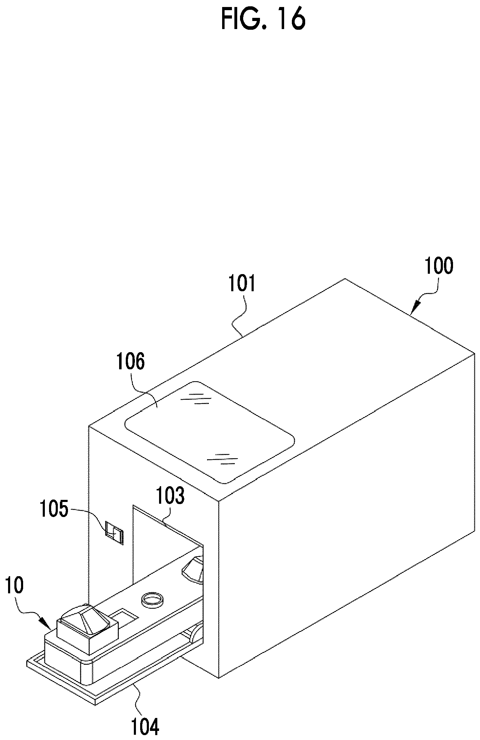

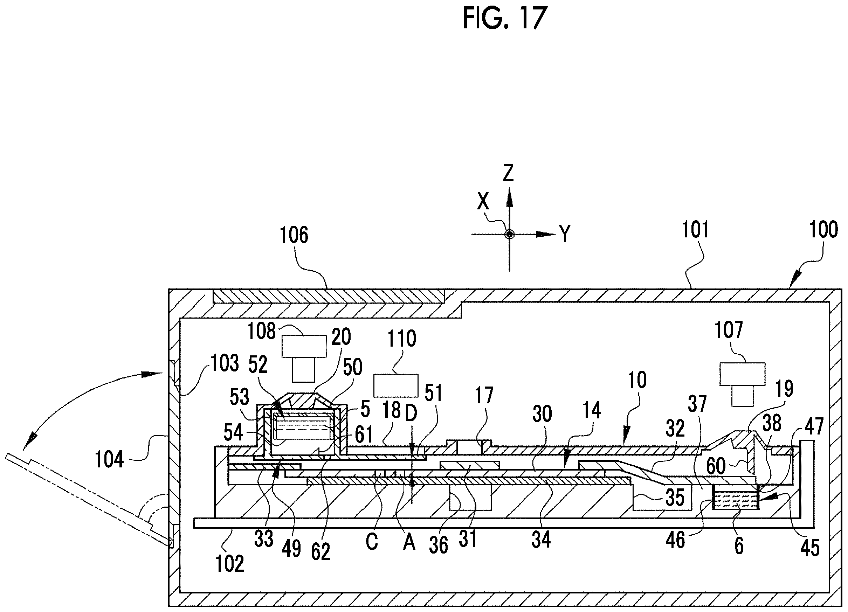

It is noted that the luminescence from the assay region A is detected by a detection unit 110 such as a spectrophotometer in an assay apparatus 100 (see Fig. 16 and Fig. 17) described later.

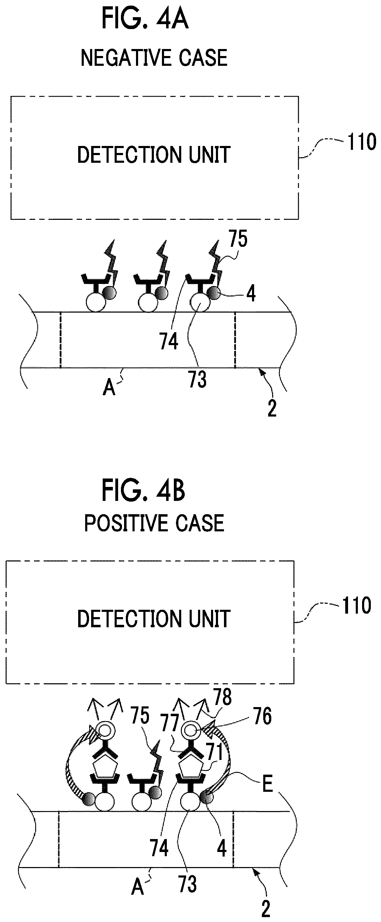

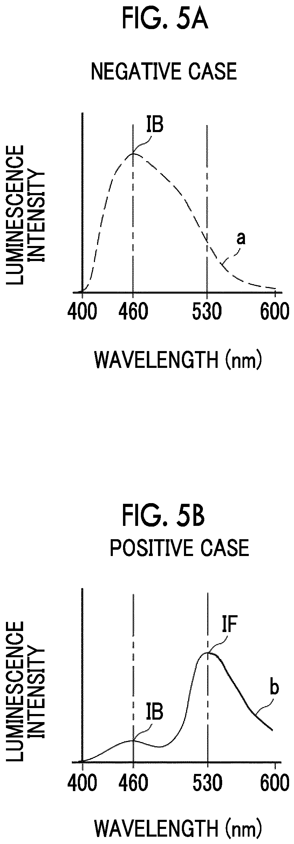

Here, the luminescence from the assay region A in each of a positive case and a negative case will be described with reference to Figs. 4A, 4B, 5A and 5B.

Figs. 4A and 4B are schematic views for describing each of (a) the luminescence from the assay region A in a case where the sample is negative (a negative case) and (b) the luminescence from the assay region A in a case where the sample is positive (a positive case). Figs. 5A and 5B show (a) an emission spectrum from the assay region A in the negative case and (b) an emission spectrum from the assay region A in the positive case. It is noted that as an example, the light 75 emitted by the luminescent protein 73 is assumed to be blue light having a peak wavelength of 460 nm, and the fluorescence emitted by the fluorescent substance 76 is assumed to be green light having a peak wavelength of 530 nm.

In a case where the sample is negative, the test substance 71 is not contained in the sample. Accordingly, the test substance 71 is not captured by the binding substance for capturing 74 of the assay region A as shown in Fig. 4A, and as a result, the fluorescent substance 76 is not captured either. In this state, in a case where the luminescent substrate 4 is supplied to the assay region A, the luminescent substrate 4 reacts with the luminescent protein 73 to generate luminescence energy, and luminescence occurs. In this case, the light 75 of luminescent protein 73 is generated. In a case where the sample is negative, an emission spectrum a having a peak at 460 nm, shown in Fig. 5A as an example, is obtained. The emission spectrum a shown in Fig. 5A is an example of an emission spectrum in a case where the light 75 is blue light having a peak wavelength of 460 nm.

In a case where the sample is positive, the test substance 71 is captured by the binding substance for capturing 74 of the assay region A as shown in Fig. 4B, and the fluorescent substance 76 is captured through the test substance 71. In this state, in a case where the luminescent substrate 4 is supplied to the assay region A, the luminescent substrate 4 reacts with the luminescent protein 73 to generate luminescence energy E, and the fluorescence 78 is generated from the fluorescent substance 76 due to BRET. The fluorescence 78 is green light having a peak wavelength of 530 nm. It is noted that even in the positive case, the binding substance for capturing 74 which has not captured the test substance 71 is also present in the assay region A. Therefore, in a case where the luminescent protein 73 of the binding substance for capturing 74 which has not captured the fluorescent substance 76 reacts with the luminescent substrate 4, BRET does not occur, and the light 75 having a peak wavelength of 460 nm, which depends on the luminescent protein 73, is generated. As a result, the fluorescence 78 and the light 75 are simultaneously emitted from the assay region A, and from the assay region A, an emission spectrum b having peaks at wavelengths of 460 nm and 530 nm, as shown in Fig. 5B as an example, is obtained.

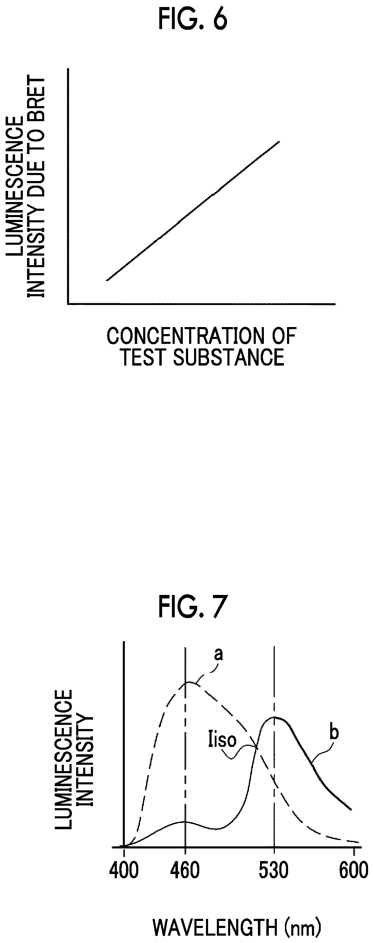

It is noted that as shown in Fig. 6, the larger the amount of the test substance 71 in the sample is, that is, the higher the concentration of the test substance 71 is, the larger the luminescence intensity from the fluorescent substance 76 due to BRET is. Then, the luminescent substrate 4 reacts with the luminescent protein 73, and the light 75 generated from the luminescent substrate 4 relatively decreases. That is, in the negative case where the test substance 71 is not present in the sample, an emission spectrum of the light 75, which has a peak wavelength at 460 nm, is obtained as shown in Fig. 5A, where the peak at 460 nm in the detected emission spectrum becomes small as the amount of the test substance 71 in the sample increases, and the peak at 530 nm becomes relatively large.

It is noted that in a case where the assay apparatus 100 (see Fig. 16) which will be described later is used, the emission spectrum a or b as shown in Figs. 5A and 5B is detected, a luminescence intensity IF of the fluorescence 78 generated due to BRET (hereinafter, referred to as a first luminescence intensity IF) and a luminescence intensity IB of the light 75 that is emitted without causing BRET to occur (hereinafter, referred to as a second luminescence intensity IB)are acquired, and a value IF/IB obtained by dividing the first luminescence intensity IF by the second luminescence intensity IB (hereinafter, referred to as a luminescence intensity ratio IF/IB) is determined, whereby it is possible to carry out a determination on whether positive or negative. For example, in a case where the luminescence intensity ratio IF/IB is larger than a predetermined threshold value, the sample is determined to be positive, and in a case where it is equal to or smaller than the threshold value, the sample is determined to be negative.

It is noted that using a luminescence intensity Iiso at the isoluminescence point at which the luminescence intensity does not change in an emission spectrum a for the negative case and in an emission spectrum b for the positive case as shown in Fig. 7 (hereinafter, referred to as a third luminescence intensity Iiso) a ratio IF/Iiso (a luminescence intensity ratio IF/Iiso) obtained by dividing the luminescence intensity IF of the fluorescence 78 generated due to BRET by the luminescence intensity Iiso at the isoluminescence point is determined as the luminescence intensity ratio to be used for the determination, whereby the determination on whether positive or negative may be carried out. For example, in a case where the luminescence intensity ratio IF/Iiso is larger than a predetermined threshold value, the sample is determined to be positive, and in a case where it is equal to or smaller than the threshold value, the sample is determined to be negative.

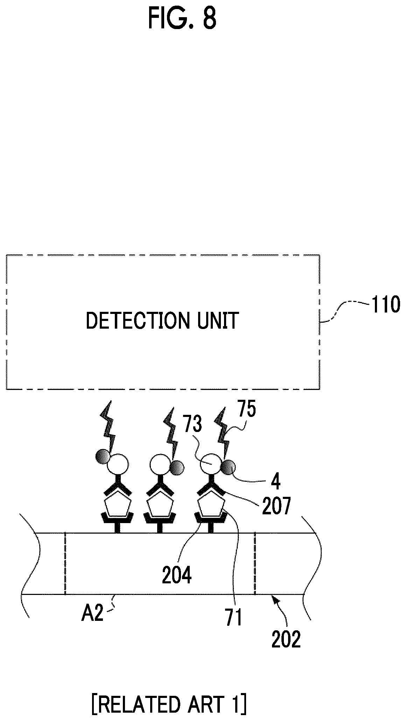

In the chemiluminescence immunochromatography which does not use BRET, which is described in JP2015-105858A described above, as shown in Fig. 8, a capture antibody 204 which is not labeled is immobilized in an assay region A2 of an assay strip 202, and a luminescent protein 73 is used as a label of a labeled antibody 207. In a case where the sample is positive, the labeled antibody 207 labeled with the luminescent protein 73 is captured through the test substance 71. The luminescent substrate 4 is supplied to the assay region A2 in this state, and thus the luminescent substrate 4 reacts with the luminescent protein 73 to emit light. In this case, the luminescent substrate 4 reacts with the luminescent protein 73, whereby the light 75 having a peak wavelength of the luminescent protein 73 is generated. In this method, in a case where the labeled antibody 207 that has non-specifically adsorbed is present, the luminescent protein 73, which is a label of the labeled antibody 207 that has non-specifically adsorbed, reacts with the luminescent substrate 4 to emit light even in a case where the labeled antibody 207 that has non-specifically adsorbed has not bound to the test substance 71. In the luminescence due to the luminescent protein 73 of the non-specifically adsorbed labeled antibody 207 in the peripheral region of the assay region A2, there is a problem that in a case where the background is increased, the S/N ratio decreases, and thus the measurement accuracy decreases.

On the other hand, the immunochromatography kit 1 according to the present embodiment includes the cartridge 3 that accommodates the assay strip 2 having the assay region A in which a sample is spread and the test substance 71 contained in the sample is captured, the binding substance for capturing 74 that is labeled with the luminescent protein 73 and immobilized on the assay region A, the labeled binding substance 77 that is labeled with the fluorescent substance 76 and supplied to the assay region A, and the luminescent substrate 4.

According to such a configuration, the energy E that is generated by the reaction between the luminescent substrate 4 and the luminescent protein 73 is transferred to the fluorescent substance 76 in a state where the labeled binding substance 77 is captured through the test substance 71, and then the fluorescence 78 is generated from the fluorescent substance 76. A phenomenon called BRET, in which this energy is transferred, occurs in a case where the luminescent protein 73 and the fluorescent substance 76 are close to each other (for example, in a state where they are positioned to each other at a distance of about 10 nm or less). As a result, in a case where the labeled binding substance 77 is not captured by the binding substance for capturing 74, BRET does not occur since the luminescent protein 73 and the fluorescent substance 76 are not close to each other. Therefore, even in a case where the labeled binding substance 77 is non-specifically adsorbed in the peripheral region of the assay region A, the fluorescent substance 76 labeled to this labeled binding substance 77 that has non-specifically adsorbed is not excited, and fluorescence is not generated. As a result, even in a case where the labeled binding substance 77 that has non-specifically adsorbed is generated in the peripheral region of the assay region A, the background does not increase, which makes it possible to carry out measurement with a high S/N ratio. That is, the immunochromatography kit 1 according to the present embodiment, which enables determination by a measurement using BRET, makes it possible to suppress the background as compared with the above-described chemiluminescence immunochromatographic method in the related art, in which this BRET is not used, and thus the measurement accuracy is high. Only in a case where the binding substance for capturing 74 labeled with the luminescent protein 73 is immobilized in the assay region A of the assay strip 2 and the binding substance for capturing 74 labeled with the luminescent protein 73 reacts with the luminescent substrate 4, a luminescence reaction occurs. In addition, only in a case where the labeled binding substance 77 is captured by this binding substance for capturing 74 through the test substance 71, the fluorescence 78 from the fluorescent substance 76 due to BRET is detected. As a result, the luminescence in the portion other than the assay region A is suppressed, the background can be suppressed, and the measurement accuracy can be improved.

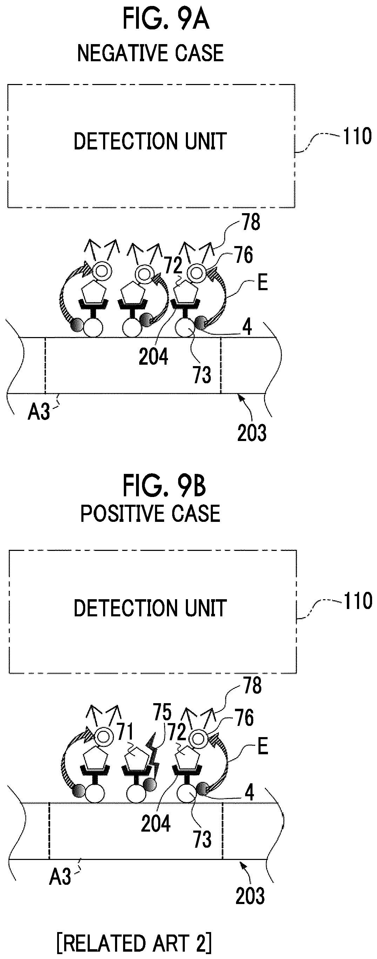

In addition, in the chemiluminescence immunochromatography, which uses the competition method described in JP2018-522221A described above and uses BRET, the capture antibody 204 labeled with the luminescent protein 73 is immobilized in the assay region A3 of the assay strip 203 similarly to the technique according to the present disclosure. In the competition method disclosed in JP2015-105858A, a labeled pseudo antigen 72 labeled with the fluorescent substance 76 and the luminescent substrate 4 are spread on the assay strip 203 together with a sample. Figs. 9A and 9B are schematic views for describing each of (a) the luminescence from an assay region A3 in a case where the sample is negative (a negative case) and (b) the luminescence from the assay region A3 in a case where the sample is positive (a positive case). In the negative case as shown Fig. 9A, the labeled pseudo antigen 72 is captured by the capture antibody 204 in the assay region A3, and the luminescence energy E generated by the reaction between the luminescent substrate 4 and the luminescent protein 73 is transferred to the fluorescent substance 76, whereby the fluorescence 78 is generated from the fluorescent substance 76. In addition, in the positive case as shown Fig. 9B, the labeled pseudo antigen 72 and the test substance 71 are competitively captured by the capture antibody 204 in the assay region A3. Since the test substance 71 does not have a label, the luminescence energy E generated by the reaction between the luminescent protein 73, which labels the capture antibody 204 that has captured the test substance 71, and the luminescent substrate 4 becomes the light 75 without being transferred to the fluorescent substance 76, and luminescence occurs. As a result, in the positive case, the fluorescence 78 from the fluorescent substance 76 due to BRET decreases, and the light 75 generated by the reaction between the luminescent protein 73 and the luminescent substrate 4 increases.

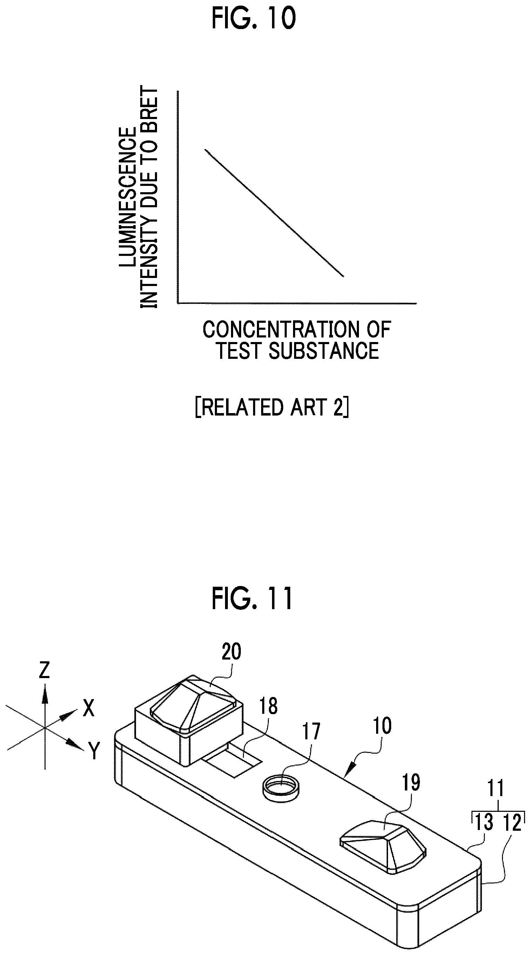

That is, according to the competition method, as shown in Fig. 10, the luminescence intensity of the fluorescence 78 generated from the fluorescent substance 76 due to BRET is highest in a state where the test substance 71 is not present in the sample, and the signal intensity of the fluorescence 78 generated from the fluorescent substance 76 due to BRET decreases as the amount of the test substance 71 increases (as the test substance concentration increases). In a case where an antigen, which is the test substance 71, is not present in the sample, the labeled pseudo antigen 72 binds to the capture antibody 204. In a case where the test substance 71 is not present, the amount of the antigen that is bound to the capture antibody 204 or the binding manner between the antigen and capture antibody 204 fluctuates due to the fluctuation of the fluid velocity, reaction temperature, or the like, which is accompanied by the variation of the sample amount, and as a result, a variation in signal intensity is likely to occur. There is a problem that the measurement accuracy is low in a case where the test substance 71 has a very small amount and in a case where the difference from the baseline, where the test substance 71 is not present is unlikely to be observed, and the test substance 71 has a very small amount.

On the other hand, the immunochromatography kit 1 according to the present embodiment uses a sandwich method in which in the assay region A of the assay strip 2, the test substance 71 is sandwiched between the binding substance for capturing 74 and the labeled binding substance 77. Therefore, in a case where the amount of the test substance in the sample is small, the measurement accuracy is high as compared with the measurement accuracy obtained by the BRET in the related art which uses the competition method. In the sandwich method, the signal intensity of the fluorescence generated from the fluorescent substance due to BRET is zero in a case where the test substance 71 is not present, and the signal intensity thereof increases as the amount of the test substance 71 increases (see Fig. 6). Therefore, it is easy to detect a change in signal intensity in a case where the test substance 71 has a very small amount, and the measurement accuracy is high in a case where the test substance 71 has a very small amount.

In the present embodiment, the washing solution 6 is provided and supplied to the assay region A after the sample and the labeled binding substance 77 are supplied to the assay region A and before the luminescent substrate 4 is supplied to the assay region. Since the labeled antibody 207 that has non-specifically adsorbed can be removed by the washing solution 6, the measurement accuracy can be improved.

The immunochromatography kit 1 according to the above-described embodiment includes a luminescent substrate solution 5 containing the washing solution 6 and the luminescent substrate 4, separately from the cartridge. However, in the immunochromatography kit according to the present disclosure, the cartridge may encompass not only the assay strip but also at least one of a luminescent substrate solution holding part 52 that encompasses the luminescent substrate solution 5 containing the luminescent substrate 4 or a washing solution holding part 45 that encompasses the washing solution 6.

Next, a cartridge 10 and the assay apparatus 100 of a design change example will be described. It is noted that the same constitutional elements as those of the cartridge 3 described with reference to Figs. 1 to 4 are denoted by the same reference numerals, and detailed description thereof will be omitted.

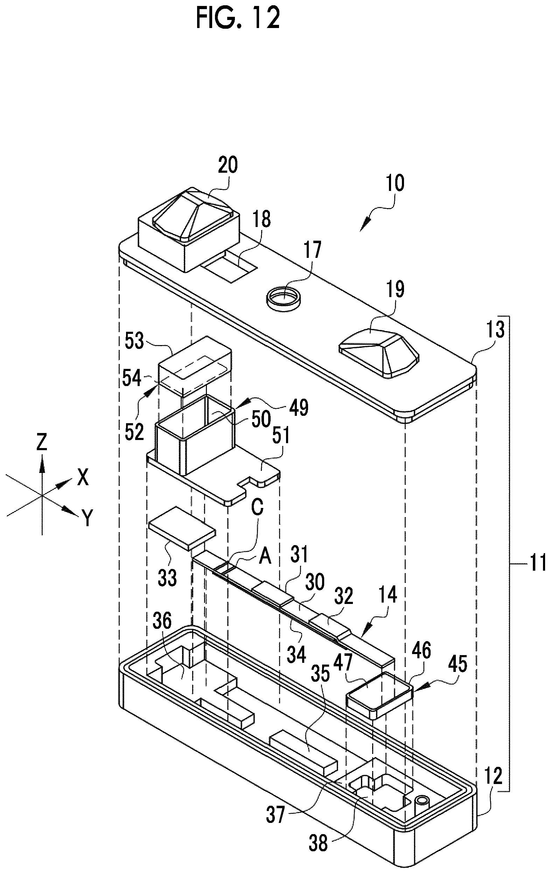

The cartridge 10 of the design change example, shown in Fig. 11 and Fig. 12, has a case 11 having an elongated rectangular parallelepiped shape. The case 11 is composed of a case main body 12 and a cover member 13. The case main body 12 is a box having an accommodation space that is surrounded by a bottom plate having an elongated rectangular plate shape and four side plates standing vertically from four sides of the bottom plate. An assay strip 14 having the assay region A is accommodated in the accommodation space of the case main body 12.

The cover member 13 has an elongated rectangular plate shape similarly to the bottom plate of the case main body 12 and functions as a lid that covers the accommodation space of the case main body 12.

A sample dropping port 17 and an observation window 18 are formed in the cover member 13. In addition, a first pressing operation part 19 and a second pressing operation part 20 are provided in the cover member 13. The sample dropping port 17, the observation window 18, the first pressing operation part 19, and the second pressing operation part 20 are integrally formed.

The sample dropping port 17 is a round hole to which the sample solution 70 is dropped, and it has a boss shape in which the edge protrudes from the surface. The sample dropping port 17 is formed in a central part of the cover member 13.

The observation window 18 is a rectangular opening for observing, from the outside, the assay region A or the like of the assay strip 14. The observation window 18 is formed between the sample dropping port 17 and the second pressing operation part 20.

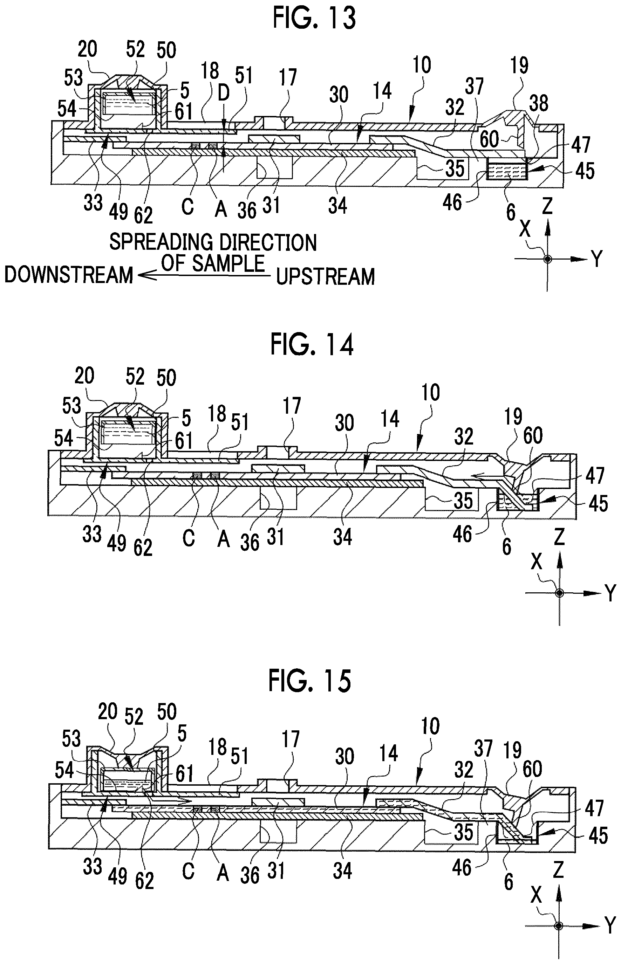

The first pressing operation part 19 is provided in one end part of the cover member 13 in the Y direction. The first pressing operation part 19 is subjected to a pressing operation by a user in a case where the washing solution 6 (see Fig. 14) is supplied to the assay strip 14. The second pressing operation part 20 is provided in the other end part of the cover member 13 in the Y direction, which is on a side opposite to the first pressing operation part 19. The second pressing operation part 20 is subjected to a pressing operation by a user in a case where the luminescent substrate solution 5 (see Fig. 15) is supplied to the assay strip 14.

The assay strip 14 has a liquid feeding pad 32 in addition to the carrier 30, the label holding pad 31, the absorption pad 33, and the back pressure-sensitive adhesive sheet 34. In addition to the assay region A, a control region C is provided on one end side of the carrier 30 in the Y direction. In a case where a direction from the label holding pad 31 toward the assay region A or the like is defined as a spreading direction of the sample (see Fig. 13), the assay region A is positioned on the upstream side of the control region C in the spreading direction. It is noted that although the assay region A and the control region C are hatched in Fig. 12, this hatching is carried out for convenience of the description and thus does not indicate that the respective regions A and C have undergone color development. The same applies to the following Fig. 13 to Fig. 15, Fig. 17, and the like.

The liquid feeding pad 32 is attached to one end of the carrier 30, which is on a side opposite to the other end of the carrier 30 having the assay region A or the like. The liquid feeding pad 32 is formed from a porous material similarly to the carrier 30 and the like, and it feeds the washing solution 6 to the carrier 30 by the capillary action.

The back pressure-sensitive adhesive sheet 34 and then the assay strip 14 are placed on protruding support tables 35 and 36 which are formed in the accommodation space of the case main body 12. The support table 35 is provided in a central part of the case main body 12. The support table 36 is provided in one end part of the case main body 12 on the downstream side in the spreading direction of the sample solution 70. The support tables 35 and 36 are at the same height.

A protruding support table 37 is also formed in the other end part of the case main body 12 on the upstream side in the spreading direction of the sample solution 70. The support table 37 is at the same height as the support tables 35 and 36. The liquid feeding pad 32 is placed on the support table 37.

A first housing part 38 having a recessed shape is formed in the support table 37. The first housing part 38 is provided at a position where it faces one end of the liquid feeding pad 32, which is on a side opposite to the other end attached to the carrier 30. The washing solution holding part 45 is accommodated in the first housing part 38.

The washing solution holding part 45 is composed of a container 46 that has an opening on one surface and a seal 47 that liquid-tightly covers the opening of the container 46. The container 46 is formed from, for example, a resin material. The washing solution 6 is stored in the inside of the container 46. The seal 47 is, for example, an aluminum sheet, and it can be easily broken by a sharp blade or the like. The washing solution holding part 45 is accommodated in the first housing part 38 with the seal 47 being faced upward so that the seal 47 faces the other end of the liquid feeding pad 32.

A multifunctional member 49 is disposed in the upper part of the support table 36. The multifunctional member 49 is formed from a transparent resin material, for example, an acrylic resin. The multifunctional member 49 is a member in which a second housing part 50 and a flow channel forming part 51 are integrally provided. The second housing part 50 is a box of which the upper surface is open, and the luminescent substrate solution holding part 52 is accommodated in the inside of the second housing part 50. The flow channel forming part 51 is a flat plate that extends from the bottom part of the second housing part 50 in the Y direction. The flow channel forming part 51 extends to the front of the label holding pad 31 and covers the upper part of the assay region A and the control region C of the carrier 30. A spacing D is provided between the carrier 30 and the flow channel forming part 51 (see Fig. 13). The spacing D is, for example, in a range of 0.01 mm to 1 mm. Since the spacing D is provided between the carrier 30 and the flow channel forming part 51 in this way, a flow channel of the luminescent substrate solution 5 is ensured.

The luminescent substrate solution holding part 52 is composed of a container 53 that has an opening on one surface and a seal 54 that liquid-tightly covers the opening of the container 53. The container 53 is formed from, for example, a resin material. The luminescent substrate solution 5 is stored in the inside of the container 53. The seal 54 is, for example, an aluminum sheet, and it can be easily broken by a sharp blade or the like. The luminescent substrate solution holding part 52 is accommodated in the second housing part 50 with the seal 54 being faced downward so that the seal 54 faces the assay strip 2.

As shown in Fig. 13 as an example, a first breaking protrusion 60 is provided in the first pressing operation part 19. In addition, the luminescent substrate solution holding part 52 is attached to an inner surface of the second pressing operation part 20. A second breaking protrusion 61 and a supply port 62 are provided at the bottom part of the second housing part 50. The distal end of each of first breaking protrusion 60 and the second breaking protrusion 61 is sharp.

As shown in Fig. 14 as an example, in a case where the first pressing operation part 19 is subjected to a pressing operation, the first breaking protrusion 60 abuts on the other end of the liquid feeding pad 32 and pushes the other end of the liquid feeding pad 32 toward the seal 47 of the washing solution holding part 45. The seal 47 is broken due to the pushing of the other end of the liquid feeding pad 32 by the first breaking protrusion 60, and the other end of the liquid feeding pad 32 falls into the container 46 to be immersed in the washing solution 6. The washing solution 6 is spread from the other end of the liquid feeding pad 32 toward the carrier 30 by the capillary action. The first pressing operation part 19 is maintained in a crushed state even after being crushed by the pressing operation. Therefore, the spreading of the washing solution 6 is continued until substantially the entire washing solution 6 is sucked up to the liquid feeding pad 32.

As shown in Fig. 15 as an example, in a case where the second pressing operation part 20 is subjected to a pressing operation, the luminescent substrate solution holding part 52 is moved downward in the second housing part 50 and reaches the bottom part of the second housing part 50 having the second breaking protrusion 61. Then, the seal 54 of the luminescent substrate solution holding part 52 is broken by the second breaking protrusion 61. The luminescent substrate solution 5 stored in the luminescent substrate solution holding part 52 flows from the broken portion of the seal 54 to the supply port 62. Further, it flows through the flow channel ensured by the spacing D (see Fig. 13) and then is supplied to the carrier 30.

The control region C contains a control binding substance 79 labeled with the luminescent protein 73. The control binding substance 79 specifically binds to the labeled binding substance 77. As a result, the fluorescent substance 76 is captured in the control region C. Some labeled binding substances 77 may not bind to the test substance 71. Such a labeled binding substance 77 reaches the control region C without being captured in the assay region A and then is captured in the control region C. In a case where the fluorescent substance 76 is captured through the labeled binding substance 77, the luminescent protein 73 and the fluorescent substance 76 are positioned close to each other in the control region C. In a case where the luminescent substrate 4 is spread in this state, BRET occurs in the control region C. The luminescence from the fluorescent substance 76 due to BRET makes it possible to confirm that the sample solution 70 has been sufficiently spread on the carrier 30 and the spreading of the sample solution 70 has been completed.

The control binding substance 79 may be the test substance 71 itself or may be a compound having a moiety that is recognized by the labeled binding substance 77. Examples of the compound having a moiety that is recognized by the labeled binding substance 77 (the compound that specifically binds to the labeled binding substance 77) include such a compound that is obtained by bonding a derivative of the test substance 71 to a protein. For example, in a case where the test substance 71 is an influenza A type virus or a biomarker thereof, it is possible to use, as the control binding substance 79, an anti-mouse IgG antibody (manufactured by Fujifilm Wako Pure Chemical Corporation, product name: anti-mouse IgG (H + L), rabbit F(ab')2, product code: 566-70621).

The procedure of the immunochromatography assay using the cartridge 10 of the above-described design change example is the same as the procedure of the assay using the cartridge 10, which has been described with reference to Fig. 3.

The cartridge 10 of the above-described design change example includes, in the cartridge 10, the luminescent substrate solution holding part 52 that encompasses the luminescent substrate solution 5 containing the luminescent substrate 4. Since the luminescent substrate solution 5 is provided in the cartridge 10 in the immunochromatography kit 1, the work of, for example, measuring the luminescent substrate solution 5 from a container such as an ampule or a vial and supplying the luminescent substrate solution 5 to the assay region A, decreases. The present cartridge 10 includes the second pressing operation part 20, and thus the luminescent substrate solution 5 can be supplied to the assay strip 14 in a case where the second pressing operation part 20 is pressed down, whereby a simple assay can be realized.

The cartridge 10 includes the washing solution holding part 45 that encompasses the washing solution 6 in the cartridge 10. Since the washing solution 6 is provided in the cartridge 10 in the immunochromatography kit 1, the work of, for example, measuring the luminescent substrate solution 5 from a container such as an ampule or a vial and supplying the luminescent substrate solution 5 to the assay region A, decreases. The present cartridge 10 includes the first pressing operation part 19, and thus the washing solution 6 can be supplied to the assay strip 14 in a case where the first pressing operation part 19 is pressed down, whereby a simple assay can be realized.

As described above, the first pressing operation part 19 and the second pressing operation part 20 of the cartridge 10 can be subjected to a pressing operation by a user. However, according to the assay apparatus 100 described below, it is also possible to carry out, in the assay apparatus 100, the pressing of the first pressing operation part 19 and the second pressing operation part 20.

Next, the configuration of the assay apparatus 100 that carries out the immunochromatography assay will be described.

As shown in Fig. 16 and Fig. 17 as an example, the assay apparatus 100 includes a housing 101 having a rectangular parallelepiped shape. A loading unit 102 having an L-shaped tray, into which the cartridge 10 is loaded, is provided in the housing 101.

A loading port 103 for the cartridge 10 is formed on a front surface of the housing 101. In addition, an openable and closable lid 104 that covers the loading port 103 is attached to the front surface of the housing 101. Further, a power switch 105 of the assay apparatus 100 is disposed on the front surface of the housing 101.

A touch panel display 106 is attached to an upper surface of the housing 101. The touch panel display 106 receives an operation instruction such as a user's instruction to start the immunochromatography assay (hereinafter, referred to as an assay start instruction). In addition, the touch panel display 106 displays information such as a determination result.

A user opens the lid 104 to expose the loading port 103 and loads the cartridge 10, in which the sample solution 70 has been spotted, into the loading unit 102 through the loading port 103. Then, the user closes the lid 104 and operates the touch panel display 106 to input an assay start instruction.

As shown in Fig. 17, the assay apparatus 100 includes, in the housing 101, a washing solution supply mechanism 107, a luminescent substrate supply mechanism 108, and a detection unit 110.

The washing solution supply mechanism 107 is a mechanism for starting the supply of the washing solution 6 from the washing solution holding part 45 to the carrier 30. As the washing solution supply mechanism 107, for example, an actuator such as a solenoid equipped with an electromagnet and a plunger movable with respect to the electromagnet is used. For example, the plunger moves, and as a result, the plunger abuts on the first pressing operation part 19 to press the first pressing operation part 19. The washing solution supply mechanism 107 is disposed at a position where it faces the first pressing operation part 19 of the loaded cartridge 10.

The washing solution supply mechanism 107 is a pressing mechanism that applies a pressing force to the first pressing operation part 19 from the outside by pressing the first pressing operation part 19 of the cartridge 10. In a case where a pressing force is applied to the first pressing operation part 19 by the washing solution supply mechanism 107, the washing solution 6 is supplied from the washing solution holding part 45 to the carrier 30 by the above-described action.

The luminescent substrate supply mechanism 108 is a mechanism for starting the supply of the luminescent substrate solution 5 from the luminescent substrate solution holding part 52 to the carrier 30. The luminescent substrate supply mechanism 108 also uses an actuator such as a solenoid similarly to the washing solution supply mechanism 107. The luminescent substrate supply mechanism 108 is disposed at a position where it faces the second pressing operation part 20 of the loaded cartridge 10. The luminescent substrate supply mechanism 108 is a pressing mechanism that applies a pressing force to the second pressing operation part 20 from the outside by pressing the second pressing operation part 20 of the cartridge 10. In a case where a pressing force is applied to the second pressing operation part 20 by the luminescent substrate supply mechanism 108, the luminescent substrate 4 is supplied from the luminescent substrate solution holding part 52 to the carrier 30 by the above-described action.

The detection unit 110 is disposed at a position where it faces the observation window 18 of the case 11. The detection unit 110 detects the luminescence from the assay region A and the control region C. Specifically, the detection unit 110 is a spectrophotometer that is capable of detecting the light 75 generated by the reaction between the luminescent protein 73 and the luminescent substrate 4 and the fluorescence 78 from the fluorescent substance 76. According to the spectrophotometer, it is possible to detect the light intensity for each wavelength.

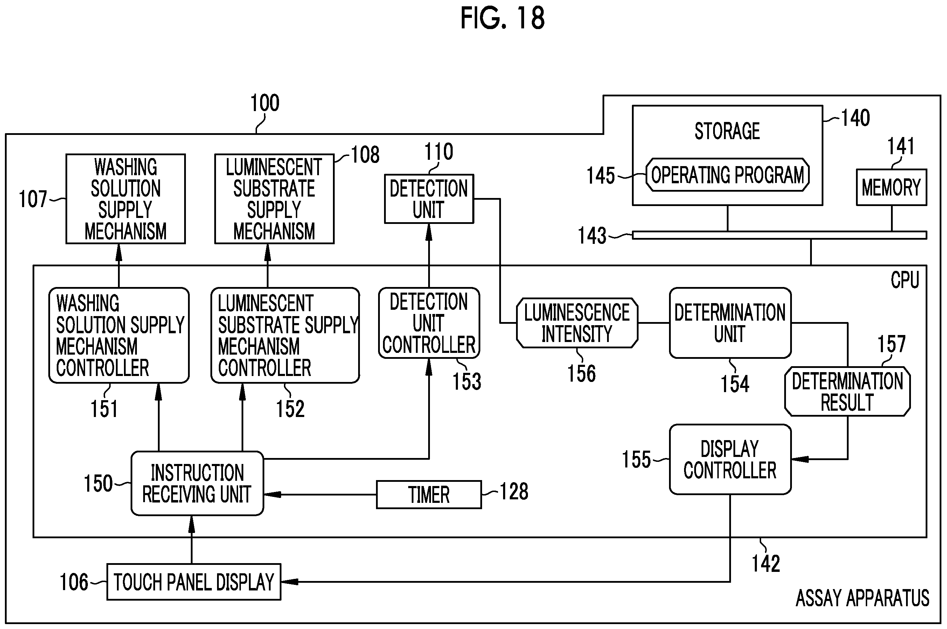

As shown in Fig. 18 as an example, the assay apparatus 100 includes a storage 140, a memory 141, and a central processing unit (CPU) 142. The storage 140, the memory 141, and the CPU 142 are connected to each other through a bus line 143. The storage 140, the memory 141, the CPU 142, and the bus line 143 constitute a computer.

The storage 140 is, for example, a hard disk drive or a solid state drive. The memory 141 is a work memory that is used in a case where the CPU 142 executes processing. The CPU 142 loads a program stored in the storage 140 into the memory 141 and executes processing according to the program. As a result, the CPU 142 carries out the overall control of the respective units of the assay apparatus 100.

An operation program 145 is stored in the storage 140. The operation program 145 is an application program for making a computer function as the assay apparatus 100 according to the present disclosure. It is noted that in addition to the operation program 145, the storage 140 also stores information necessary for identifying the color development state of the assay region A.

In a case where the operation program 145 is started, the CPU 142 cooperates with the memory 141 and the like to function as an instruction receiving unit 150, a washing solution supply mechanism controller 151, a luminescent substrate supply mechanism controller 152, a detection unit controller 153, a determination unit 154, and a display controller 155.

The instruction receiving unit 150 receives various operation instructions from a user through the touch panel display 106. For example, the instruction receiving unit 150 receives an assay start instruction. In a case of having received an assay start instruction, the instruction receiving unit 150 manages the time on the basis of the timer 128 and outputs at each timing a control start trigger signal to the washing solution supply mechanism controller 151, the luminescent substrate supply mechanism controller 152, and the detection unit controller 153. After the assay start instruction, a control start trigger signal is output to the washing solution supply mechanism controller 151 at a time point t1 after a predetermined time has elapsed. Thereafter, a control start trigger signal is output to the luminescent substrate supply mechanism controller 152 at a time point t2 after a predetermined time has elapsed, and further, a control start trigger signal is output to the detection unit controller 153 at a subsequent time point t3. As a result, the supply of the washing solution 6, the supply of the luminescent substrate 4, and the light detection are executed at an optimum timing. In a case of controlling the timing of the supply of the luminescent substrate and the time taken from the supply of the luminescent substrate 4 to the measurement, it is possible to realize a more accurate measurement.

The washing solution supply mechanism controller 151 operates the washing solution supply mechanism 107 to control the first pressing operation part 19 to be pressed. The washing solution supply mechanism controller 151 operates the washing solution supply mechanism 107 to supply the washing solution 6 to the assay region A after the sample and the labeled binding substance 77 are supplied to the assay region A and before the luminescent substrate 4 is supplied to the assay region.

The luminescent substrate supply mechanism controller 152 operates the luminescent substrate supply mechanism 108 to control the second pressing operation part 20 to be pressed. The luminescent substrate supply mechanism controller 152 operates the luminescent substrate supply mechanism 108 to supply the luminescent substrate 4 to the assay region A after the sample and the labeled binding substance 77 are supplied to the assay region A.

The detection unit controller 153 controls the operation of the detection unit 110. The detection unit controller 153 drives the detection unit 110 to detect the luminescence from the assay region A. Specifically, an emission spectrum in a wavelength range including the light 75 and the fluorescence 78 is acquired. The detection unit 110 outputs an acquired emission spectrum 156 to the determination unit 154.

The determination unit 154 carries out a determination on whether the sample is positive or negative based on the emission spectrum 156 acquired from the detection unit 110. The determination unit 154 carries out a determination on whether the sample is positive or negative based on the luminescence intensity of the fluorescence 78 generated from the fluorescent substance 76 due to BRET. In a case of carrying out the determination, not only the luminescence intensity of the fluorescence 78 but also the luminescence intensity of the light 75 generated by the reaction between the luminescent protein 73 and the luminescent substrate 4, which is not associated with BRET, may be used. The determination unit 154 outputs a determination result 157 to the display controller 155.

Here, a more specific example of the processing in the determination unit 154 will be described. The determination unit 154 acquires, for example, an emission spectrum a or b as shown in Figs. 5A and 5B, as the emission spectrum 156 from the detection unit 110. The determination unit 154 acquires, from the emission spectrum a or b, the first luminescence intensity IF that is the luminescence intensity of the fluorescence 78 generated due to BRET and the second luminescence intensity IB that is the luminescence intensity of the light 75 emitted without causing the BRET, and determines the luminescence intensity ratio IF/IB, which is a value obtained by dividing the first luminescence intensity IF by the second luminescence intensity IB. Then, a determination on whether positive or negative is carried out based on the luminescence intensity ratio IF/IB. For example, in a case where the luminescence intensity ratio IF/IB is larger than a predetermined threshold value, the sample is determined to be positive, and in a case where it is equal to or smaller than the threshold value, the sample is determined to be negative.

It is noted that instead of acquiring the second luminescence intensity IB from the emission spectrum a or b, the determination unit 154 may acquire the third luminescence intensity Iiso, which is a luminescence intensity at the isoluminescence point at which the luminescence intensity does not change in the emission spectrum a for the negative case and in the emission spectrum b for the positive case as shown in Fig. 7, and may determine the luminescence intensity ratio IF/Iiso, which is a value obtained by dividing the first luminescence intensity IF by the third luminescence intensity Iiso, thereby carrying out a determination on whether positive or negative. For example, in a case where the luminescence intensity ratio IF/Iiso is larger than a predetermined threshold value, the sample is determined to be positive, and in a case where it is equal to or smaller than the threshold value, the sample is determined to be negative.

The display controller 155 controls display of various types of information on the touch panel display 106. For example, the display controller 155 displays the determination result 157 from the determination unit 154, on the touch panel display 106.

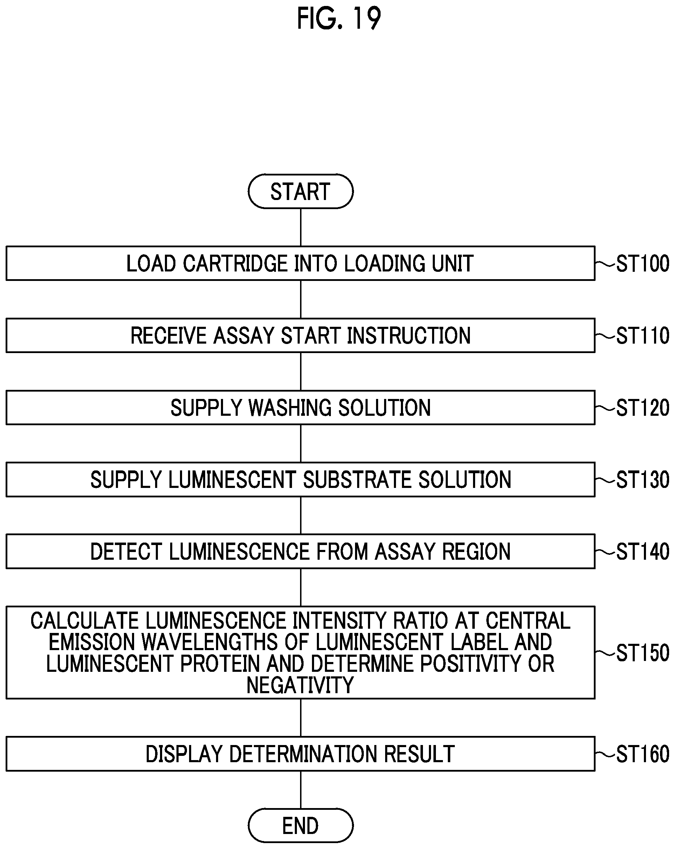

Next, an action with the configuration described above will be described with reference to the flowchart shown in Fig. 19 as an example. First, a user carries out a step of spotting the sample solution 70 on the cartridge 10. Then, the power switch 105 is operated to allow a power source to turn on the assay apparatus 100, and the operation program 145 is started. In a case where the operation program 145 is started, the CPU 142 of the assay apparatus 100 functions as an instruction receiving unit 150, a washing solution supply mechanism controller 151, a luminescent substrate supply mechanism controller 152, a detection unit controller 153, a determination unit 154, and a display controller 155, as shown in Fig. 18.

The user opens the lid 104 and loads the cartridge 10 into the loading unit 102 through the loading port 103 (a step ST100). Then, the user closes the lid 104.

Subsequently, the user operates the touch panel display 106 and inputs an assay start instruction. The instruction receiving unit 150 receives the assay start instruction (a step ST110). Then, a control signal is output, at each timing from the start of the assay, from the instruction receiving unit 150 to the washing solution supply mechanism controller 151, the luminescent substrate supply mechanism controller 152, and the detection unit controller 153.

First, with a time of receiving the instruction being as a reference, the washing solution supply mechanism 107 is operated to press the first pressing operation part 19 under the control of the washing solution supply mechanism controller 151 at the time point t1. As a result, the first pressing operation part 19 is pressed down, and as shown in Fig. 14, the liquid feeding pad 32 is immersed in the washing solution 6, and the washing solution 6 is supplied to the assay strip 14 (a step ST120).

Thereafter, at the time point t2 after a predetermined time has elapsed from the time point 11, the luminescent substrate supply mechanism 108 is operated to press the second pressing operation part 20 under the control of the luminescent substrate supply mechanism controller 152. As a result, the second pressing operation part 20 is pressed down, and the luminescent substrate solution holding part 52 is pushed down, whereby the seal 54 of the luminescent substrate solution holding part 52 is broken by the second breaking protrusion 61, as shown in Fig. 15. As a result, the luminescent substrate solution 5 flows from the supply port 62 and is supplied to the assay strip 14 (a step ST130).

Next, at the time point t3 after a predetermined time has elapsed from the time point t2, the detection unit 110 is driven under the control of the detection unit controller 153 to detect the luminescence intensity (the emission spectrum 156) in a wavelength range including the peak wavelength of the fluorescence of the fluorescent substance 76 and the peak wavelength of the luminescent protein 73 (a step ST140).

The emission spectrum 156 is analyzed in the determination unit 154, and the luminescence intensity ratio is calculated to determine the positivity or the negativity (a step ST150). The determination result 157 is output from the determination unit 154 to the display controller 155.

A message indicating the determination result 157 is displayed on the touch panel display 106 under the control of the display controller 155 (a step ST160). In this way, the immunochromatography assay for one cartridge 10 ends.

As described above, the assay apparatus 100 includes the loading unit 102 into which the cartridge 10 is attachably and detachably loaded, the detection unit 110 that detects the intensity of the luminescence from the fluorescent substance 76 in the assay region A, and the processor (here, the CPU 142) that is configured to carry out a determination on whether the sample is positive or negative based on the intensity of the luminescence acquired from the detection unit 110.

According to the assay apparatus 100 having the above-described configuration, the positivity or the negativity is determined based on the luminescence intensity of the fluorescence 78 from the fluorescent substance 76 due to BRET, and thus it is possible to obtain a highly accurate measurement result.

In addition, the assay apparatus 100 according to the present embodiment includes the luminescent substrate supply mechanism 108 that supplies the luminescent substrate 4 to the assay region A, where the processor (here, the CPU 142) is configured to supply the luminescent substrate 4 to the assay region A by the luminescent substrate supply mechanism 108 after the sample and the labeled binding substance 77 are supplied to the assay region A. That is, the luminescent substrate 4 is supplied in a state where the sample and the labeled binding substance 77 are specifically bound to the assay region A. The luminescence reaction between the luminescent substrate 4 and the luminescent protein 73 proceeds irreversibly by the contact between the luminescent substrate 4 and the luminescent protein 73, and the luminescence intensity decreases with the elapse of time. Therefore, as compared with a case where the luminescent substrate 4 is mixed with a sample in advance and then incubated, an effect of suppressing the reduction of the luminescence intensity in a case of detecting luminescence from the assay region A can be obtained.

In the assay apparatus 100 according to the present embodiment, the first luminescence intensity IF, which is the intensity of the luminescence (the fluorescence 78) from the fluorescent substance 76 in the assay region A, is detected, and the intensity of the light 75 emitted from the luminescent protein 73 is detected as the second luminescence intensity IB, and it is possible to carry out a determination on whether positive or negative based on the luminescence intensity ratio IF/IB. Since the luminescence intensity ratio is used, it is possible to obtain a determination result having a high accuracy as compared with a case of carrying out a determination using only the first luminescence intensity IF.

In addition, in the assay apparatus 100 according to the present embodiment, the intensity of the luminescence (the fluorescence 78) from the fluorescent substance 76 in the assay region A is detected as the first luminescence intensity IF, the intensity of the luminescence at the isoluminescence point, which is present in a wavelength range between a peak wavelength of the luminescence from the fluorescent substance 76 and a peak wavelength of the luminescence which occurs by the reaction between the luminescent substrate 4 and the luminescent protein 73 is detected as the third luminescence intensity Iiso, and it is possible to carry out a determination on whether positive or negative based on the luminescence intensity ratio IF/Iiso. Since the luminescence intensity ratio is used, it is possible to obtain a determination result having a high accuracy as compared with a case of carrying out a determination using only the first luminescence intensity IF.

In the above-described embodiments, various processors to be described below can be used as a hardware structure of a processing unit, which carries out various types of processing. The various processors include, for example, the CPU 142 which is a general-purpose processor executing software (the operation program 145) to function as various processing units, a programmable logic device (PLD), such as a field programmable gate array (FPGA), which is a processor of which the circuit configuration can be changed after manufacture, and/or a dedicated electric circuit, such as an application specific integrated circuit (ASIC), which is a processor having a dedicated circuit configuration designed to execute a specific process.

One processing unit may be composed of one of these various processors or may be composed of a combination of two or more processors of the same type or different types (for example, a combination of a plurality of FPGAs and/or a combination of a CPU and an FPGA). Further, a plurality of processing units may be composed of one processor.

As an example in which the plurality of processing units is composed of one processor, first, as represented by a computer such as a client and a server, there is a configuration in which one processor is composed of a combination of one or more CPUs and software and the processor functions as a plurality of processing units. Secondly, as represented by a system on chip (SoC) or the like, there is a configuration in which a processor that realizes the functions of the entire system including a plurality of processing units by one integrated circuit (IC) chip is used. As described above, one or more of the above various processors are used to constitute the hardware structure of the various processing units.

Further, more specifically, an electric circuit (circuitry) in which circuit elements, such as semiconductor elements, are combined can be used as the hardware structure of the various processors.

It is noted that in the above description, the description has been made for the case of carrying out a determination on whether the sample is positive or negative by using the assay apparatus 100. However, as described above, since the color of the luminescence from the assay region A changes between the negative case and the positive case, a user can visually recognize the luminescence from the assay region A to carry out a determination on whether positive or negative, without using the assay apparatus 100.

### Examples

Hereinafter, Examples and Comparative Examples according to the technology of the present disclosure will be described. An influenza A type antigen was used as a test substance. Assay strips of Examples 1 to 3 were such strips that were for a use application to the chemiluminescence immunochromatography assay which used a sandwich method and used BRET. The assay strips of Example 2 and Example 3 were the same.

### <Example 1>

### (1) Assay strip

### (Carrier)

A porous carrier (hereinafter, referred to as a carrier) having a size of 60 mm × 300 mm was prepared. As a porous carrier, a nitrocellulose membrane (having a plastic lining, HiFlow Plus HF135 (capillary flow rate = 135 seconds/cm, manufactured by Millipore Corporation)) was used. A line-shaped assay region was formed along a length direction of 300 mm at a position of 15 mm from one end (hereinafter, referred to as a downstream end) of carrier in a length direction of 60 mm.

### (Formation of assay region)

An assay region was formed by immobilizing a binding substance for capturing on the assay region.

As a luminescent protein, Luciferase (Nanoluc (registered trade name): manufactured by Promega Corporation, peak wavelength: 460 nm) was used. As a binding substance for capturing, an anti-influenza A type monoclonal antibody (Anti-influenza ASPTN-5 7307, manufactured by Medix Biochemica) was used. A solution containing a binding substance for capturing consisting of an anti-influenza A type monoclonal antibody labeled with Luciferase was prepared to be 1.5 mg/mL. After applying the prepared solution containing the binding substance for capturing onto the assay region, the carrier was dried for 30 minutes. Next, the dried carrier was immersed in a vat containing 500 mL of a borate buffer (pH 8.5) of 50 mmol/L, which contained a blocking solution (0.5% by mass casein (derived from milk, product number: 030-01505, manufactured by FUJIFILM Wako Pure Chemical Corporation)), and allowed to stand for 30 minutes as it was. Then, the carrier was taken out, and the carrier was immersed in 500 mL of a washing and stabilizing solution (a 50 mmol/L Tris-HCl (pH 7.5) buffer containing 0.5% by mass sucrose and 0.05% by mass sodium cholate) prepared in another vat, and allowed to stand as it was for 30 minutes. Then, the carrier was taken out from the solution and dried in an environment of 25°C for 24 hours.

The capture antibody was immobilized on the assay region by the above step to form an assay region.

### (Label holding pad)

A label holding pad that held a labeled binding substance was prepared.

A glass fiber pad (Glass Fiber Conjugate Pad, manufactured by Millipore Corporation) cut into a size of 5 mm × 300 mm was prepared.

A green fluorescent protein mNeogreen (manufactured by Allele Biotechnology and Pharmaceuticals, Inc., peak wavelength: 517 nm) was used as a fluorescent substance, and an influenza A type antibody was used as a labeled binding substance (here, as a labeled antibody). The influenza A type antibody-fluorescent substance composite body was diluted with water so that the absorbance at 506 nm was 0.8 in a case where a concentration of a Tris-HCl buffer (pH 8.2) was 20 mmol/L, a concentration of PEG (Mw: 20,000) was 0.05% by mass, a concentration of sucrose was 5% by mass, and an optical path length was set to 10 mm, whereby a coating liquid of the anti-influenza A type fragmented antibody-fluorescent substance composite body was obtained. 0.1 mL of this coating liquid was uniformly applied per one pad of the above-described pad and dried under reduced pressure for 24 hours. In this way, a label holding pad that held a labeled binding substance (here, a labeled antibody) labeled with a fluorescent substance was obtained.

### (Back pressure-sensitive adhesive sheet)

A pressure-sensitive adhesive sheet (manufactured by NIPPN Techno Cluster, Inc.) having a size of 60 mm × 300 nm was prepared as a back pressure-sensitive adhesive sheet.

### (Liquid feeding pad)

A glass fiber pad (Glass Fiber Conjugate Pad, manufactured by Millipore Corporation) cut into a size of 25 mm × 300 mm was used as a liquid feeding pad.

### (Absorption pad)

A glass fiber pad (glass filter paper, manufactured by Advantech Co., Ltd.) cut into a length of 12 mm and a width of 10 mm was used as an absorption pad.

Using the above components, an assay strip was prepared according to the following procedure.

The carrier on which the assay region had been formed was attached to the back pressure-sensitive adhesive sheet. Next, a double-sided tape (Nitto Denko Corporation) having a width of 3 mm was fixed at a position of 26 mm from the downstream end of the carrier in the direction of 60 mm. Then, the label holding pad was fixed to the carrier so that the downstream end of the double-sided tape and the downstream end of the label holding pad overlapped each other. The liquid feeding pad was attached to the upstream side of the carrier opposite to the downstream end so that the liquid feeding pad and the carrier overlapped each other by 7 mm. The sheet-shaped member prepared in this way was cut parallelly to the length direction of 60 mm to have a width of 5 mm with a guillotine type cutter (CM4000, manufactured by NIPPN Techno Cluster, Inc.).

In this way, 60 strips having a width of 5 mm and a length of 60 mm were obtained. One absorption pad was disposed at the downstream end of one strip to be overlapped each other, thereby obtaining an assay strip.

### (2) Spreading solution

The following spreading solutions were prepared.

### (Reference spreading solution)

A reference spreading solution was prepared such that casein was 0.05% by mass, Tween (registered trade name) 40 was 0.5% by mass, and a Tricine buffer solution (pH 8.5) was 200 mmol/L.

### (Spreading solution-1)

A simulated positive sample (BD Flu Examan Control A + B -) manufactured by Becton, Dickinson and Company) was mixed with a reference spreading solution so that the antigen amount of thereof was equivalent to twice the minimum detection sensitivity (detection limit) of the influenza A virus antigen, which was described in a package insert of "FUJI DRI-CHEM IMMUNO AG cartridge FluAB", whereby a spreading solution-1 was obtained.

### (Spreading solution-2)

A simulated positive sample was mixed with a reference sample solution so that the amount of the simulated positive sample (the antigen amount) was 300 times that of the spreading solution-1, whereby a spreading solution-2 was obtained.

### (3) Luminescent substrate solution

A solution containing a 20 × 10⁻⁶ M luminescent substrate (Furimazine, Promega Corporation) was used as a luminescent substrate solution. The luminescent substrate solution to be used in one assay was set to 100 µL.

### (Procedure of immunochromatography assay)

1) A sample solution is spotted on the label holding pad.
2) 10 minutes after the spotting of the sample solution, the luminescent substrate solution is supplied to the porous carrier.
3) 20 seconds after the supply of the luminescent substrate solution, the luminescence from the assay region is measured with a spectrophotometer. The luminescence intensity ratio (517 nm/460 nm) of the intensity of the fluorescence (peak wavelength: 517 nm) from the fluorescent substance to the intensity of the luminescence (peak wavelength: 460 nm) generated by the reaction between the luminescent protein and the luminescent substrate is calculated.

The following three patterns of assays were carried out: a reference assay in which the reference spreading solution containing no antigen is used as a sample solution; an assay 1 in which the spreading solution-1 with a low-concentration antigen, which has a small amount of antigen, is used as a sample solution; and an assay 2 in which the spreading solution-2 with a high-concentration antigen, which has a large amount of antigen, is used as a sample solution.

Each of the above-described assays using the reference spreading solution, the spreading solution-1, and the spreading solution-2 was carried out 10 times. The preparation of the assay strip and the preparation of the spreading solution were carried out for each measurement.

### <Example 2>

In the preparation of the label holding pad of Example 1, a method of preparing a labeled antibody was as follows.

The fact that the anti-influenza A type monoclonal antibody is used as an antibody is common to Example 2 and Example 1. As a fluorescent substance, a fluorescent dye (peak wavelength: 525 nm) of a fluorescent antibody labeling kit (Thermo Fisher Scientific Inc.) was used. The anti-influenza A type monoclonal antibody was labeled with the fluorescent dye according to the procedure of the fluorescent antibody labeling kit to obtain a labeled binding substance (here, a labeled antibody) labeled with a fluorescent substance. An assay strip of Example 2 was prepared in the same manner as in Example 1, except for the above points.

The spreading solution, the luminescent substrate solution, and the assay procedure were the same as in Example 1.

### <Example 3>

As an assay strip, a spreading solution, and a luminescent substrate solution, the same ones as in Example 1 were used. However, in Example 3, a washing step using a washing solution was incorporated into the assay procedure of Example 1. After spotting the sample solution of the above-described assay procedure 1), the liquid feeding pad was immersed in the washing solution, and the washing solution was fed for 10 minutes. By this step, the labeled antibody that had been non-specifically adsorbed was washed. Thereafter, the above-described assay procedures 2) and 3) were carried out. The washing solution is supplied to the liquid feeding pad to feed the washing solution for 10 minutes. The liquid feeding pad is immersed in a pot accommodating 200 µL of the washing solution, and the washing solution is spread in the carrier by a capillary force. After washing, by this step, the labeled antibody that has not specifically adsorbed (has non-specifically adsorbed), the supply of the luminescent substrate solution of the procedure 2), and the measurement of the luminescence intensity of the procedure 3) are carried out.

The same assay and evaluation as in Example 1 were carried out except that the assay procedure was changed as described above.

Assay strips of Comparative Example 1 and Comparative Example 2 were such strips that were for a use application to the chemiluminescence immunochromatography which used a competition method and used BRET.

### <Comparative Example 1>

### (Preparation of label holding pad)

A label holding pad that held a labeled pseudo antigen was prepared.

### (Labeled pseudo antigen)

As the labeled pseudo antigen, an anti-influenza A type pseudo antigen labeled with a fluorescent substance was used.

A glass fiber pad (Glass Fiber Conjugate Pad, manufactured by Millipore Corporation) cut into a size of 5 mm × 300 mm was prepared.

A green fluorescent protein mNeogreen (peak wavelength: 517 nm) was used as a fluorescent substance, and an influenza A type pseudo antigen was used as a labeled binding substance (here, as a labeled pseudo antigen).

The anti-influenza A type pseudo antigen labeled with a fluorescent substance was diluted by using a 20 mM Tris-HCl buffer (pH 8.2), 5% by mass sucrose, and water, and a coating liquid was prepared so that the absorbance thereof was 0.8 at 506 nm in a case where the optical path length was set to 10 mm. 0.1 mL of this coating liquid was uniformly applied per one pad of the above-described pad to obtain a label holding pad that held a labeled pseudo antigen labeled with a fluorescent substance. An assay strip of Comparative Example 1 was prepared in the same manner as in Example 1, except for the above points.

### <Comparative Example 2>

An assay strip of Comparative Example 2 was such a strip that was for a use application to the chemiluminescence immunochromatography assay which did not use BRET.

### (1) Assay strip

### (Formation of assay region)

As a binding substance for capturing to be immobilized in the assay region, an anti-influenza A type antigen (a labeled pseudo antigen) labeled with a luminescent protein was used. As the luminescent protein, Luciferase (Nanoluc (registered trade name): manufactured by Promega Corporation, luminescence wavelength: 460 nm) was used. A solution containing a binding substance for capturing consisting of an anti-influenza A type antigen labeled with Luciferase was prepared to be 1.5 mg/mL. An assay region was formed on the carrier in the same manner as in Example 1, except for this point.

### (Label holding pad)

A label holding pad that held a labeled binding substance (here, a labeled antibody) was prepared.

A glass fiber pad (Glass Fiber Conjugate Pad, manufactured by Millipore Corporation) cut into a size of 5 mm × 300 mm was prepared.

A green fluorescent protein mNeogreen (manufactured by Allele Biotechnology and Pharmaceuticals, Inc., peak wavelength: 517 nm) was used as a fluorescent substance, and an anti-influenza A type monoclonal antibody (Anti-influenza ASPTN-5 7307, manufactured by Medix Biochemica) was used as a labeled antibody.

A solution of a labeled antibody consisting of the anti-influenza A type monoclonal antibody labeled with a fluorescent protein was diluted using a 20 mM Tris-HCl buffer (pH 8.2), 5% by mass sucrose, and water, and then was prepared so that the absorbance thereof was 0.8 at 506 nm in a case where the optical path length was set to 10 mm. 0.1 mL of this coating liquid was uniformly applied per one glass fiber pad of the above-described pad to obtain a holding pad that held a labeled antibody labeled with a fluorescent substance.

An assay strip of Comparative Example 1 was prepared in the same manner as in Example 2, except for the above points. The labeled pseudo antigen is immobilized in the assay region of the assay strip of Comparative Example 2, and the labeled antibody that has not bound to the antigen which is a test substance is captured by the labeled pseudo antigen. In Comparative Example 2, the antigen, which is a test substance, and the labeled pseudo antigen competitively bind to the labeled antibody.

The spreading solution, the luminescent substrate solution, and the assay procedure were the same as in Example 1.

### (Evaluation)

Each of Examples and Comparative Examples was evaluated as follows.

For the reference assay, the average value of the luminescence intensity ratios obtained by carrying measurements 10 times was calculated. That is, an average value R0av of the luminescence intensity ratios R0 obtained by the measurement using a spreading solution containing no antigen was calculated. This average value R0av was used as a negative reference. Regarding the 10 times of assays for each of the luminescence intensity ratio R1 obtained in the assay using the spreading solution-1 containing an antigen at a low concentration and the luminescence intensity ratio R2 measured in the assay using the spreading solution-2 containing an antigen at a high concentration, the evaluation was carried out as follows according to the number of times where there was a significant difference with respect to ROav, which is the negative reference. The results are listed in Table 1.
A: 10 times
B: 8 or 9 times
C: 6 or 7 times
D: 5 times or less

**[Table 1]**

| | Antigen concentration (high) | Antigen concentration (low) |
|---|---|---|
| Example 1 | A | B |
| Example 2 | A | B |
| Example 3 | A | A |
| Comparative Example 1 | A | D |
| Comparative Example 2 | A | D |

As shown in Table 1, in a case where the antigen concentration was high, a significant difference was confirmed with respect to the average value from the reference assay in all the assays for an antigen concentration of 0, including Comparative Examples. On the other hand, in a case where the antigen concentration was low, it was revealed that the antigen can be detected with good reproducibility in a case where the assay strip that was applied to the assay according to the sandwich method of Examples 1 to 3 was used, as compared with a case where the assay strip applied to the assay according to the competition method of Comparative Examples 1 and 2 is used. In addition, from the comparison between Examples 1 and 3, it was shown that in a case of including a washing step in the assay procedure, it is possible to reliably detect a sample even in a case where the antigen concentration is low.

The disclosure of JP2021-159997 filed on September 29, 2021 is incorporated in the present specification by reference in its entirety.

All documents, patent applications, and technical standards described in the present specification are incorporated herein by reference, to the same extent as in the case where each of the documents, patent applications, and technical standards is specifically and individually described.

## Claims

1. An immunochromatography kit comprising:
a cartridge that accommodates an assay strip in which a sample is spread and which has an assay region where a test substance contained in the sample is captured;
a binding substance for capturing that specifically binds to the test substance, where the binding substance for capturing is labeled with a luminescent protein and immobilized on the assay region;
a labeled binding substance that specifically binds to the test substance, where the labeled binding substance is labeled with a fluorescent substance and supplied to the assay region; and
a luminescent substrate that is supplied to the assay region, where the luminescent substrate causes a phenomenon of bioluminescence resonance energy transfer, in which the luminescent substrate reacts with the luminescent protein to generate energy, and the energy is transferred to the fluorescent substance in a state where the labeled binding substance is captured by the binding substance for capturing through the test substance.

2. The immunochromatography kit according to claim 1,
wherein the luminescent protein is a luminescent protein that causes blue luminescence to occur, and
the fluorescent substance is a green fluorescent dye or a green fluorescent protein, which emits green fluorescence having a quantum yield of 0.7 or more.

3. The immunochromatography kit according to claim 1,
wherein the luminescent protein is a blue luminescent protein that causes blue luminescence to occur, and
the fluorescent substance has a peak wavelength of 530 nm or more.

4. The immunochromatography kit according to any one of claims 1 to 3, further comprising:
a sample collection tool for collecting the sample.

5. The immunochromatography kit according to any one of claims 1 to 4, further comprising:
an instruction manual that describes an assay procedure of supplying the sample and the labeled binding substance to the assay region and then supplying the luminescent substrate to the assay region.

6. The immunochromatography kit according to any one of claims 1 to 5,
wherein a label holding pad containing the labeled binding substance is disposed at a supply position of the sample on the assay strip.

7. The immunochromatography kit according to any one of claims 1 to 6,
wherein a luminescent substrate solution holding part that encompasses a luminescent substrate solution containing the luminescent substrate is provided in the cartridge.

8. The immunochromatography kit according to any one of claims 1 to 7, further comprising:
a washing solution that is supplied to the assay region after supplying the sample and the labeled binding substance to the assay region and before supplying the luminescent substrate to the assay region, where the washing solution is used to wash the labeled binding substance that has non-specifically adsorbed in the assay region.

9. The immunochromatography kit according to claim 8,
wherein a washing solution holding part that encompasses the washing solution is provided in the cartridge.

10. An assay apparatus comprising:
a loading unit in which the cartridge in the immunochromatography kit according to any one of claims 1 to 9 is attachably and detachably loaded;
a detection unit that detects an intensity of luminescence from the fluorescent substance in the assay region; and
a processor that is configured to determine whether the sample is positive or negative based on the intensity of the luminescence acquired from the detection unit.

11. The assay apparatus according to claim 10, further comprising:
a luminescent substrate supply mechanism that supplies the luminescent substrate to the assay region,
wherein the processor is configured to supply the luminescent substrate to the assay region by the luminescent substrate supply mechanism after the sample and the labeled binding substance are supplied to the assay region.

12. The assay apparatus according to claim 11, further comprising:
a washing solution supply mechanism that supplies the washing solution to the assay region,
wherein the cartridge in the immunochromatography kit according to claim 9 is loaded into the loading unit, and
the processor is configured to supply the washing solution to the assay region by the washing solution supply mechanism after supplying the sample and the labeled binding substance to the assay region and before supplying the luminescent substrate to the assay region.

13. The assay apparatus according to any one of claims 10 to 12,
wherein the detection unit detects the intensity of the luminescence from the fluorescent substance in the assay region as a first luminescence intensity and detects an intensity of luminescence from the luminescent protein as a second luminescence intensity, and
the processor is configured to acquire the first luminescence intensity and the second luminescence intensity from the detection unit and carry out the determination using a value obtained by dividing the first luminescence intensity by the second luminescence intensity.

14. The assay apparatus according to any one of claims 10 to 12,
wherein the detection unit detects, as a first luminescence intensity, the intensity of the luminescence from the fluorescent substance in the assay region and detects, as a third luminescence intensity, an intensity of luminescence at an isoluminescence point, which is present in a wavelength range between a peak wavelength of the luminescence from the fluorescent substance and a peak wavelength of the luminescence from the luminescent protein, and
the processor is configured to acquire the first luminescence intensity and the third luminescence intensity from the detection unit and carry out the determination using a value obtained by dividing the first luminescence intensity by the third luminescence intensity.
